(19) Europäisches Patentamt
European Patent Office
Office européen des brevets

(11) **EP 2 088 977 B1**

(12) **EUROPÄISCHE PATENTSCHRIFT**

(45) Veröffentlichungstag und Bekanntmachung des Hinweises auf die Patenterteilung:
**11.09.2013 Patentblatt 2013/37**

(21) Anmeldenummer: **07819732.4**

(22) Anmeldetag: **09.11.2007**

(51) Int Cl.:
***A61F 9/008*** *(2006.01)*

(86) Internationale Anmeldenummer:
**PCT/EP2007/009739**

(87) Internationale Veröffentlichungsnummer:
**WO 2008/055697 (15.05.2008 Gazette 2008/20)**

(54) **BEHANDLUNGSVORRICHTUNG ZUR OPERATIVEN FEHLSICHTIGKEITSKORREKTUR EINES AUGES UND VERFAHREN ZUM ERZEUGEN VON STEUERDATEN ZUR OPERATIVEN FEHLSICHTIGKEITSKORREKTUR EINES AUGES**

DEVICE FOR CORRECTIVE OPHTHALMOLOGIC SURGERY AND METHOD FOR GENERATING CONTROL DATA FOR CORRECTIVE OPHTHALMOLOGIC SURGERY

DISPOSITIF DE TRAITEMENT DE CORRECTION D'UN DÉFAUT VISUEL DE L'OEIL PAR OPÉRATION ET PROCÉDÉ DE PRODUCTION DE DONNÉES DE COMMANDE POUR CORRECTION D'UN DÉFAUT VISUEL DE L'OEIL

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **10.11.2006 DE 102006053120**
**10.11.2006 US 858201 P**

(43) Veröffentlichungstag der Anmeldung:
**19.08.2009 Patentblatt 2009/34**

(60) Teilanmeldung:
**12180466.0 / 2 529 712**

(73) Patentinhaber: **Carl Zeiss Meditec AG**
**07745 Jena (DE)**

(72) Erfinder:
• **BISCHOFF, Mark**
  **07749 Jena (DE)**
• **STICKER, Markus**
  **07749 Jena (DE)**
• **STOBRAWA, Gregor**
  **07743 Jena (DE)**

(74) Vertreter: **Geyer, Fehners & Partner**
**Patentanwälte**
**Perhamerstrasse 31**
**80687 München (DE)**

(56) Entgegenhaltungen:
| | |
|---|---|
| **EP-A- 1 153 584** | **WO-A-96/11655** |
| **WO-A-2005/092172** | **WO-A1-2005/011547** |
| **US-A- 6 110 166** | **US-A1- 2004 243 112** |

**Beschreibung**

Die Erfindung bezieht sich auf eine Behandlungsvorrichtung zur operativen Fehlsichtigkeitskorrektur eines Auges eines Patienten, die eine von einer Steuereinrichtung gesteuerte Lasereinrichtung aufweist, welche durch Einstrahlen von Laserstrahlung Hornhaut-Gewebe trennt, wobei die Steuereinrichtung die Lasereinrichtung zur Fokussierung der Laserstrahlung in die Hornhaut auf in einem Muster in der Hornhaut liegende Zielpunkte ansteuert und das Muster so wählt, daß damit ein Volumen in der Hornhaut isoliert ist, dessen Entfernung aus der Hornhaut die gewünschte Fehlsichtigkeitskorrektur bewirkt.

Die Erfindung bezieht sich weiter auf ein Verfahren zu Erzeugen von Steuerdaten für eine Lasereinrichtung einer Behandlungsvorrichtung zur operativen Fehlsichtigkeitskorrektur eines Auges eines Patienten, welche durch Einstrahlen fokussierter Laserstrahlung Hornhaut-Gewebe trennt, wobei die Steuerdaten im Betrieb der Behandlungsvorrichtung der Lasereinrichtung Zielpunkte für die fokussierte Laserstrahlung vorgeben, die in einem Muster in der Hornhaut so liegen, daß damit ein Volumen in der Hornhaut isoliert ist, dessen Entfernung aus der Hornhaut die gewünschte Fehlsichtigkeitskorrektur bewirkt.

Der klassische Weg zur Korrektur der Fehlsichtigkeit des menschlichen Auges ist die Brille. Mittlerweile wird jedoch auch vermehrt refraktive Chirurgie eingesetzt, die durch Veränderung der Augenhornhaut eine Fehlsichtigkeitskorrektur bewirkt. Ziel der Operationsmethoden ist es dabei, die Hornhaut gezielt zu verändern, um so die Lichtbrechung zu beeinflussen. Hierfür sind unterschiedliche Operationsmethoden bekannt. Am verbreitesten ist gegenwärtig die sogenannte Laser- Insitu- Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp. Irvine, USA, vertrieben wird. Nachdem die Lamelle gelöst und zur Seite geklappt wurde, ist bei der LASIK- Operation die Anwendung eines Excimer- Lasers vorgesehen, der das derart freigelegte Hornhautgewebe durch Ablation abträgt. Wenn auf diese Art und Weise in der Hornhaut liegendes Volumen verdampft wurde, wird die Hornhautlamelle wieder auf den ursprünglichen Platz zurückgeklappt.

Die Anwendung eines Laserkeratoms zum Freilegen der Lamelle ist vorteilhaft, da die Infektionsgefahr dadurch verringert und die Schnittqualität vergrößert ist. Insbesondere kann die Lamelle mit sehr viel konstanterer Dicke hergestellt werden. Auch ist der Schnitt potentiell glatter, was spätere optische Störungen durch diese auch nach der Operation verbleibende Grenzefläche mindert.

Bei der Erzeugung einer Schnittfläche in der Hornhaut durch Laserstrahlung laufen im Gewebe zeitlich hintereinander mehrere Prozesse ab, die durch die gepulste Laserstrahlung initiiert werden. Liegt die Leistungsdichte der Strahlung bei einem Puls über einem Schwellwert, kommt es zu einem optischen Durchbruch, der in der Hornhaut z.B. eine Plasmablase erzeugt. Die Plasmablase wächst nach Entstehen des optischen Durchbruchs durch sich ausdehnende Gase. Wird der optische Durchbruch nicht aufrechterhalten, so wird das in der Plasmablase erzeugte Gas vom umliegenden Gewebe aufgenommen, und die Blase verschwindet wieder. Es sind auch Gewebetrenneffekte möglich, die ohne Plasmablase wirken. Der Einfachheit halber werden all solche Prozesse hier unter dem Begriff "optischer Durchbruch" zusammengefaßt, d.h. dieser Begriff soll nicht nur den optischen Durchbruch sondern auch die daraus resultierenden Wirkungen in der Hornhaut einschließen.

Zur Gewebetrennung wird die Laserstrahlung gepulst angewendet, wobei die Pulslänge in der Regel unter 1 ps liegt. Dadurch wird die zur Auslösung des optischen Durchbruchs nötige Leistungsdichte für den jeweiligen Puls in einem engen räumlichen Gebiet erreicht. Die Druckschrift US 5984916 zeigt diesbezüglich deutlich, daß der räumliche Bereich des optischen Durchbruches (in diesem Fall der erzeugten Wechselwirkung) stark von der Pulsdauer abhängt. Eine hohe Fokussierung des Laserstrahls in Kombination mit den erwähnten kurzen Pulsen erlaubt es damit, den optischen Durchbruch punktgenau in der Hornhaut einzusetzen.

Zur Schnitterzeugung wird eine Serie optischer Durchbrüche an vorbestimmten Stellen so erzeugt, daß dadurch die Schnittfläche ausgebildet wird. Beim erwähnten Laserkeratom bildet die Schnittfläche die vor dem Einsatz der Laserablation abzuklappende Lamelle.

Bei der herkömmlichen LASIK-Methode wird freigelegtes Hornhautgewebe verdampft, was auch als "Schleifen" der Hornhaut mittels Laserstrahlung bezeichnet wird. Die Volumenentfernung, die für eine Fehlsichtigkeitskorrektur notwendig ist, wird dabei für jedes Flächenelement der freigelegten Hornhaut durch die Zahl der Laserpulse und deren Energie eingestellt. Man sieht deshalb in der LASIK-Methode für den Ablationslaser ein sogenanntes shot file vor, das für verschiedene Punkte auf der Augenhornhaut vorgibt, wie oft der Laserstrahl auf definierte Punkte auf der Hornhaut gerichtet werden soll und mit welcher Energie. Die Volumenentfernung wurde dabei heuristisch ermittelt, nicht zuletzt da sie sehr von der Ablationswirkung des Laserstrahls, mithin von der Wellenlänge, Fluence etc. der eingesetzten Strahlung abhängt. Auch spielt der Zustand der Augenhornhaut eine Rolle; hier ist insbesondere der Feuchtigkeitsgehalt der Augenhornhaut zu nennen.

Erfahrungswerte, die zum Schleifen der Hornhaut mittels Ablationslaserstrahlung tauglich sind, können nun für weiterentwickelte Verfahren der refraktiven Augenchirurgie, bei denen das aus der Hornhaut zu entfernende Volumen nicht durch Ablation freigelegten Hornhautgewebes entfernt wird, sondern in der Hornhaut durch eine dreidimensionale Schnittfläche isoliert wird und somit entnehmbar gemacht, nicht verwendet werden.

Die Druckschriften US 2004/243112 A1, WO2005/011547 A1 und die US 6110166 offenbaren jeweils eine Behandlungsvorrichtung zur operativen Fehlsichtigkeitskorrektur unter Einsatz von gepulster Laserstrahlung zur Trennung von Gewebeschichten. Die Druckschriften WO 2005/092172, WO 2005/011547 A1 und WO 96/11655 betreffen die Berechnung des Krümmungsradius der Augenhornhaut.

Es ist deshalb Aufgabe der vorliegenden Erfindung, eine Behandlungsvorrichtung bzw. ein Verfahren der eingangs genannten Art so auszubilden, daß die Schnittfläche präzise definiert werden kann.

Diese Aufgabe wird erfindungsgemäß mit einer Behandlungsvorrichtung gemäß Anspruch 1 gelöst. Es ist vorgesehen, daß die um das Volumen verminderte Hornhaut einen Krümmungsradius $R_{CV}{}^*$ hat, der folgender Gleichung genügt:

$$R_{CV}{}^* = 1 / ( (1/R_{CV}) + B_{BR} / ( (n_c\text{-}1) (1 - d_{HS} \bullet B_{BR}))) + F,$$

wobei $R_{CV}$ der Krümmungsradius der Hornhaut vor Entfernung des Volumens, $n_c$ die Brechkraft des Materials der Hornhaut, F ein Faktor, $B_{BR}$ die Brechkraft einer für die Fehlsichtigkeitskorrektur tauglichen Brille, sowie $d_{HS}$ der Abstand ist, in dem die Brille mit der Brechkraft $B_{BR}$ vor dem Hornhautscheitel liegen müßte, um die gewünschte Fehlsichtigkeitskorrektur mittels der Brille zu erreichen.

Diese Aufgabe wird erfindungsgemäß weiter mit einem Verfahren gemäß Anspruch 7 zum Erzeugen für Steuerdaten für eine Lasereinrichtung gelöst. Im Verfahren zum Erzeugen von Steuerdaten für eine Lasereinrichtung einer Behandlungsvorrichtung zur operativen Fehlsichtigkeitskorrektur eines Auges eines Patienten, welche durch Einstrahlen fokussierter Laserstrahlung Hornhaut-Gewebe trennt, geben die Steuerdaten im Betrieb der Behandlungsvorrichtung der Lasereinrichtung Zielpunkte für die fokussierte Laserstrahlung vor, die in einem Muster in der Hornhaut so liegen, daß damit ein Volumen in der Hornhaut isoliert ist, dessen Entfernung aus der Hornhaut die gewünschte Fehlsichtigkeitskorrektur bewirkt.

Es wird also eine Steuergröße bzw. eine Bemessungsgröße bereitgestellt, auf deren Basis das zu entfernende Volumen und damit die dieses Volumen in der Hornhaut isolierende Schnittfläche möglichst exakt berechnet werden kann. Die oben angegebene Gleichung definiert den Krümmungsradius, den die Hornhaut nach der Entnahme des durch die Behandlungsvorrichtung bzw. das Verfahren isolierten Volumens hat. Mit dieser Gleichung kann das Volumen analytisch exakt berechnet werden. Der heuristische Ansatz, wie er beim Schleifen der im LASIK-Prozeß freigelegten Hornhaut Anwendung findet, ist durch eine analytische Beschreibung der Hornhautvorderfläche, wie sie nach der Korrektur vorliegen muß, abgelöst, was eine präzise Berechnung des zu entnehmenden Volumens beim weitergebildeten ophtalmologischen chirurgischen Verfahren ermöglicht.

Die Beschreibung der Hornhautvorderflächenkrümmung nach der Korrektur geht von Fehlsichtigkeitsdaten aus, die die Brechkraft $B_{BR}$ einer für die Fehlsichtigkeitskorrektur tauglichen Brille angeben, welche in einem Abstand $d_{HS}$ vor dem Hornhautscheitel liegen muß, um die gewünschte Fehlsichtigkeitskorrektur zu erreichen. Eine Bestimmung dieser Parameter ist gängiger Standard in der Augenoptik und ermöglicht die Verwendung weitverbreiteter und seit langem eingeführter Meßeinrichtungen. Zur Berechnung des Volumens, das in der Hornhaut zu isolieren ist, ist somit auf Fehlsichtigkeitsdaten für eine übliche Brillenkorrektur zurückzugreifen.

Selbstverständlich können solche Daten auch Astigmatismuskorrekturen oder auch Korrekturen höherer Aberrationsordnungen beinhalten. Eine übliche Formel für die Brechkraft $B_{BR}$ einer Brille ist beispielsweise die in nachfolgender Figurenbeschreibung angegebene Gleichung (1).

Sie gibt den sphärischen Brechungsfehler Sph sowie den zylindrischen Brechungsfehler Cyl an, und setzt für letzteren natürlich die Kenntnis der Zylinderachse $\theta$ voraus.

Der Faktor F ist ein Maß für die optische Wirkung der Dickenabnahme der Augenhornhaut auf der Sehachse aufgrund der Entfernung des Volumens. In einer vereinfachten Berechnung kann der Faktor F gleich Null gesetzt werden. Möchte man eine genauere Berechnung, so kann F wie folgt berechnet werden:

$$F = (1 - 1/n_c) \bullet (d_C{}^* - d_C),$$

wobei $d_C$ bzw. $d_C{}^*$ die Dicke der Hornhaut vor bzw. nach Entfernung des Volumens bezeichnet und der Radius $R_{CV}{}^*$ iterativ berechnet ist, indem bei jedem iterationsschritt aus der Differenz $(R_{CV}{}^* - R_{CV})$ auf eine Dickenänderung $(d_C{}^* - d_C)$ geschlossen wird und das entsprechend daraus erhaltene Ergebnis für die Dickenänderung bei der Berechnung von $R_{CV}{}^*$ im nächsten Iterationsschritt angewendet wird. Die iterative Berechnung für F kann beispielsweise abgebrochen werden, wenn zwischen zwei Iterationsschritten für F nur noch ein Unterschied besteht, der kleiner als ein Grenzwert ist.

Bei Berücksichtigung einer zylindrischen Fehlsichtigkeit ist der Radius, den die Hornhaut nach Verminderung um das Volumen hat, natürlich eine Funktion des Zylinderwinkels, d.h. eines Winkels senkrecht zur Sehachse, wie es in der Ophtalmologie zur Beschreibung astigmatischer Fehlsichtigkeit üblich ist. Gleiches gilt natürlich für die Brechkraft der Brille, von der die Gleichung für den Radius ausgeht.

Das Volumen ist erfindungsgemäß über die Gleichung nun so bestimmt bzw. bestimmbar, daß die Hornhaut nach Entfernung des Volumens den definierten Krümmungsradius hat. Eine besonders einfach zu berechnende und vor allem auch zu realisierende Definition des Volumens begrenzt das Volumen durch eine Grenzfläche, die in eine anteriore und eine posteriore Teilfläche unterteilt ist, wobei die anteriore Teilfläche in konstantem Abstand $d_F$ zur Hornhautvorderfläche liegt. Die Begriffe "anterior" und "posterior" entsprechen der üblichen medizinischen Nomenklatur.

Durch die in konstantem Abstand zur Hornhautoberfläche liegende anteriore Teilfläche ist die Ausbildung dieser Teilfläche besonders einfach. Die posteriore Teilfläche hat natürlich zwangsläufig dann keinen konstanten Abstand zur Hornhautvorderfläche. Die optische Korrektur erfolgt durch die Formgebung der posterioren Teilfläche. Durch diesen Ansatz ist der Rechenaufwand erheblich vereinfacht, da eine sphärische Teilfläche (die anteriore Teilfläche) besonders einfach zu berechnen ist und der Rechenaufwand in der Bestimmung der posterioren Teilfläche konzentriert ist.

Überraschenderweise zeigt sich, daß bei einem derartigen Ansatz zugleich eine einfache analytische Beschreibung der posterioren Teilfläche möglich ist. Diese hat nämlich einen Krümmungsverlauf, der bis auf eine additive Konstante identisch mit dem Krümmungsverlauf der Hornhautvorderfläche nach Entnahme des Volumens sein kann. In die Konstante fließt der Abstand, den die anteriore Teilfläche von der Hornhautvorderfläche hält, ein.

Diese Ausgestaltung erlaubt es insbesondere, die posteriore Teilfläche in Zylinderkoordinaten, deren Ursprung am Durchtrittspunkt der Sehachse durch die Hornhautvorderfläche liegt, besonders einfach zu beschreiben, nämlich durch die Gleichung

$$z_L(r,\varphi) = R_L(\varphi) - (R_L^2(\varphi) - r^2)^{1/2} + d_L + d_F,$$

wobei $d_L$ eine Mindestdicke des zu entfernenden Volumens festlegt.

Das optionale Vorsehen der Mindestdicke $d_L$ kommt der späteren Entnahme des isolierten Volumens entgegen, da das Volumen dann eine gewisse Mindestdicke (z.B. am Rand) hat, nämlich den Wert $d_L$. Zugleich kann damit sichergestellt werden, daß das Volumen die Pupille des Auges überdeckt, wobei vorzugsweise die Größe der Pupille zugrundegelegt wird, die sich bei dunkelangepaßtem Auge einstellt. Durch diese Maßnahme ist sichergestellt, daß die optische Korrektur bei allen Sehbedingungen, die für den Patienten auftreten, bestmöglich ist.

Das erfindungsgemäße Verfahren zum Vorbereiten der Steuerdaten kann ohne Rückgriff auf menschliche Mitwirkung ausgeführt werden. Insbesondere kann es von einem Computer durchgeführt werden, der aus entsprechenden Vorgaben, beispielsweise aus den funktionell definierten Bahnkurven die Steuerdaten ermittelt, indem die Stützstellenwahl passend zur Verstellgeschwindigkeit der das Zielsystem zur Applikation der Steuerdaten darstellenden Lasereinrichtung ermittelt. Insbesondere ist bei der Ermittlung der Steuerdaten die Mitwirkung eines Arztes in keiner Weise erforderlich, da mit der Ermittlung der Steuerdaten noch kein therapeutischer Eingriff verbunden ist. Dieser findet erst bei der Anwendung der zuvor ermittelten Steuerdaten statt.

Die Erfindung wird nachfolgend unter Bezugnahme auf die Zeichnungen beispielshalber noch näher erläutert. In den Zeichnungen zeigt:

Fig. 1          eine Schemadarstellung einer Behandlungsvorrichtung bzw. eines Behandlungsgerätes zur Fehlsichtigkeitskorrektur,

Fig. 1a          eine Schemadarstellung hinsichtlich des Aufbaus des Behandlungsgerätes der Fig. 1,

Fig. 2          eine Prinzipdarstellung zur Einbringung gepulster Laserstrahlung in das Auge bei der Fehlsichtigkeitskorrektur mit dem Behandlungsgerät der Fig. 1,

Fig. 3          eine weitere Schemadarstellung des Behandlungsgerätes der Fig. 1,

Fig. 4          in Teilfiguren (a), (b) und (c) schematische Schnittdarstellungen zur Verdeutlichung des Korrekturbedarfes am menschlichen Auge bei Fehlsichtigkeit,

Fig. 5          eine schematische Schnittdarstellung durch die Augenhornhaut mit Darstellung eines zur Fehlsichtigkeitskorrektur zu entfernenden Volumens,

Fig. 6          ein Schnitt durch die Augenhornhaut nach Entfernung des Volumens der Fig. 5,

Fig. 7          eine Schnittdarstellung ähnlich der Fig. 5,

Fig. 8          eine schematische Schnittdarstellung durch die Augenhornhaut zur Veranschaulichung der Volumenentnahme,

Fig. 9          eine Draufsicht auf eine spiralförmige Bahnkurve, die zur Isolierung des Volumens der Fig. 5, 7 und 8 verwendet wird,

Fig. 10         eine vergrößerte Darstellung der Bahnkurve der Fig. 9,

Fig. 11         eine alternative Bahnkurve für die Korrektur auch zylindrischer Fehlsichtigkeiten,

Fig. 12         eine schematische Darstellung zur Erläuterung der Funktion eines Kontaktglases im Behandlungsgerät der Fig. 1,

Fig. 13 bis 15    schematische Darstellungen hinsichtlich der Wirkungen des Kontaktglases durch Verformung der Augenhornhaut,

Fig. 16 und 17    Schemadarstellung hinsichtlich der Approximation der das Volumen posterior begrenzenden Fläche im Falle auch zylindrischer Fehldichtigkeitskorrekturen,

Fig. 18         eine schematische Darstellung zu einer bei der Ermittlung von Steuerdaten für das Behandlungsgerät der Fig. 1 verwendeten Bildfeldkrümmungskorrektur und

Fig. 19         eine schematische Darstellung des Ablaufes bei der Vorbereitung und Durchführung einer Fehlsichtigkeitskorrektur.

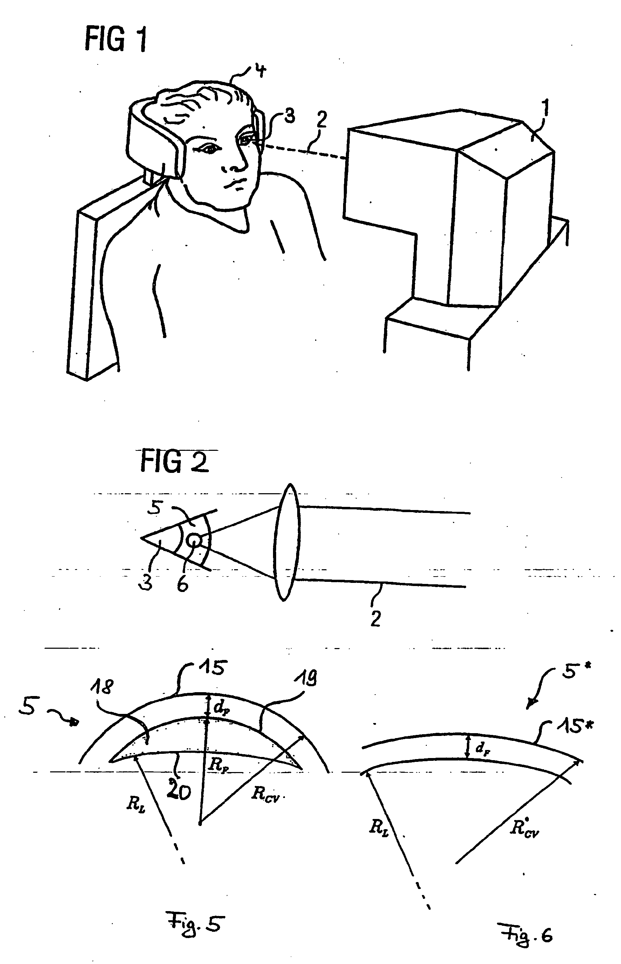

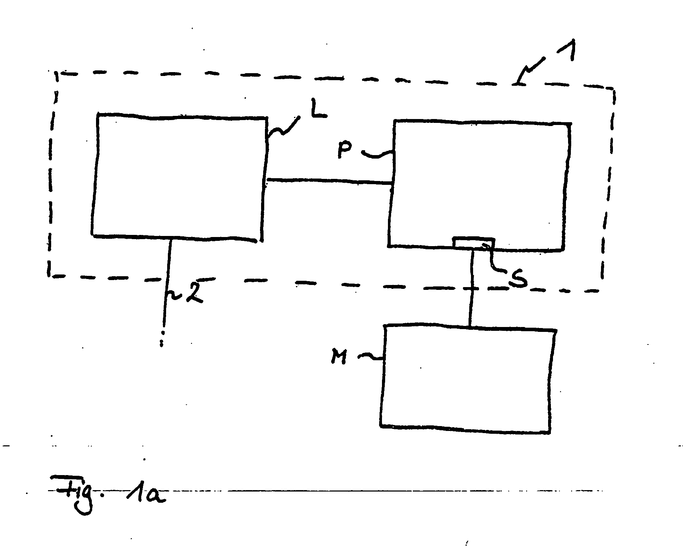

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Abberationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Meßeinrichtungen vermessen.

Figur 1a zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die für den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, daß weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Meßdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Meßeinrichtung M, die das Auge des Patienten 4 zuvor vermessen hat. Natürlich kann die Meßeinrichtung M auf beliebige Art und Weise die entsprechenden Meß- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Meßeinrichtung M mit der Behandlungseinrichtung 1 hinsichtlich der Datenübertragung, die in einer Variante verwendet werden kann, hat den Vorteil, daß die Verwendung falscher Meß- und Fehlsichtigkeitsdaten mit größtmöglicher Sicherheit ausgeschlossen ist. Dies gilt insbesondere dann, wenn die Überführung des Patienten von der Meßeinrichtung M bzw. den Meßeinrichtungen zur Lasereinrichtung L mittels einer (in der Figur nicht dargestellten) Lagerungseinrichtung erfolgt, die mit der Meßeinrichtung M bzw. der Lasereinrichtung L so zusammenwirkt, daß die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Meßeinrichtung M zur Lasereinrichtung L kann dabei zugleich auch die Übertragung der Meß-und Fehtsiehtigkeitsdaten an die Behandlungsvorrichtung 1 erfolgen.

Es ist vorzugsweise durch geeignete Mittel sichergestellt, daß die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

Die Wirkungsweise des Laserstrahls 2 ist in Figur 2 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus, der einen Spot 6 überdeckt und in dem die Laserstrahlungsenergiedichte so hoch ist, daß in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 2 schematisch angedeutete Plasmablase initiiert. Dadurch wird mittels dieses Laserpulses in der Hornhaut 5 Gewebe getrennt. Bei Entstehung einer Plasmablase umfaßt die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch von jedem Laserpuls erzeugt wird, muß die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, daß mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, wesentlich ist lediglich, daß dazu gepulste Behandlungs- Laserstrahlung 2 verwendet wird. Beispielsweise kann ein Behandlungsgerät 1 verwendet werden, wie sie in der WO 2004/032810 A2 beschrieben ist. Wesentlich ist weiter, daß eine Vielzahl von Laserpulsfoki im Gewebe eine Schnittfläche ausbildet, deren Form vom Muster abhängt, mit dem die Laserpulsfoki im Gewebe angeordnet sind/ werden. Das Muster gibt Zielpunkte für die Fokuslage vor, an denen ein oder mehrere Laserpuls (e) abgegeben wird (werden) , und definiert die Form und Lage der Schnittfläche. Für die nachfolgend erläuterten Verfahren und Vorrichtungen ist das Muster der Zielpunkte von Bedeutung und wird noch näher beschrieben werden.

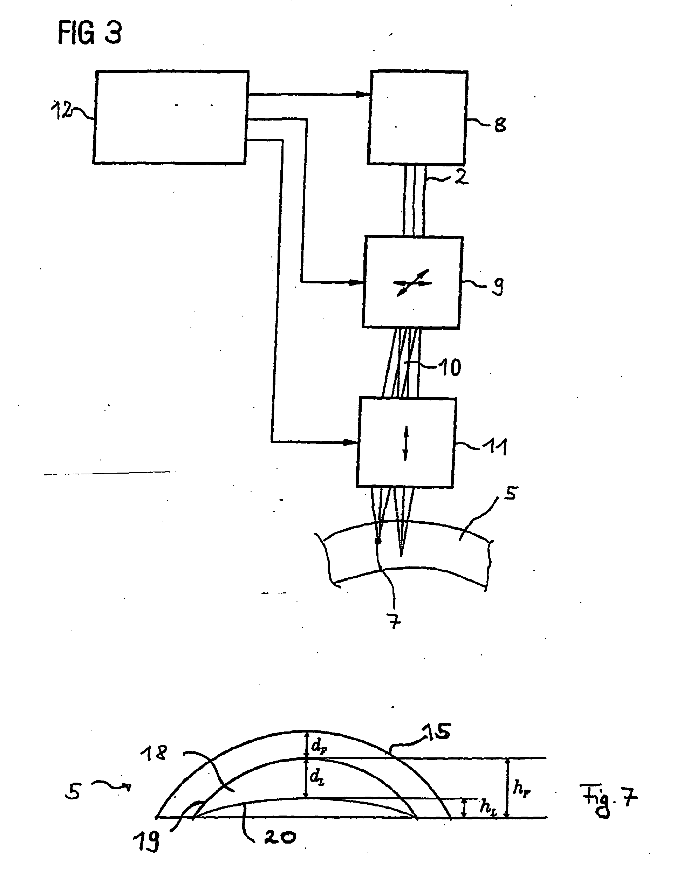

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, daß damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

In Figur 3 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so daß zur Schnittflächenerzeugung an verschiedenen Stellen fokussiert Energie aus Laserstrahlungspulsen in das Gewebe der Hornhaut 5 eintragen wird. Die Laserstrahlung 2 wird von einem Laser 8 als gepulste Strahlung bereitgestellt. Ein xy-Scanner 9, der in einer Variante durch zwei im wesentlichen orthogonal ablenkende Galvanometerspiegel realisiert ist, lenkt den vom Laser 8 kommenden Laserstrahl zweidimensional ab, so daß nach dem xy-Scanner 9 ein abgelenkter Laserstrahl 10 vorliegt. Der xy-Scanner 9 bewirkt somit eine Verstellung der Lage des Fokus 7 im wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, daß die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, daß eine dreidimensionale Beschreibung der Lage des Fokus 7 in der Hornhaut 5 auch durch andere Koordinatensysteme erfolgen kann, insbesondere muß es sich nicht um ein rechtwinkliges Koordinatensystem handeln. Daß der xy-Scanner 9 um zueinander rechtwinklige Achsen ablenkt ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-geradlinige Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 3 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so däß die Lage des Fokus 7 in der Hornhaut 5 so verstellt wird, daß letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefaßt. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muß eine Vorplanung des operativen Eingriffes dahingehend erfolgen, daß die Steuerdaten für das Behandlungsgerät 1 ermittelt werden, deren Anwendung dann eine für den Patienten 4 optimale Fehlsichtigkeitskorrektur erreicht.

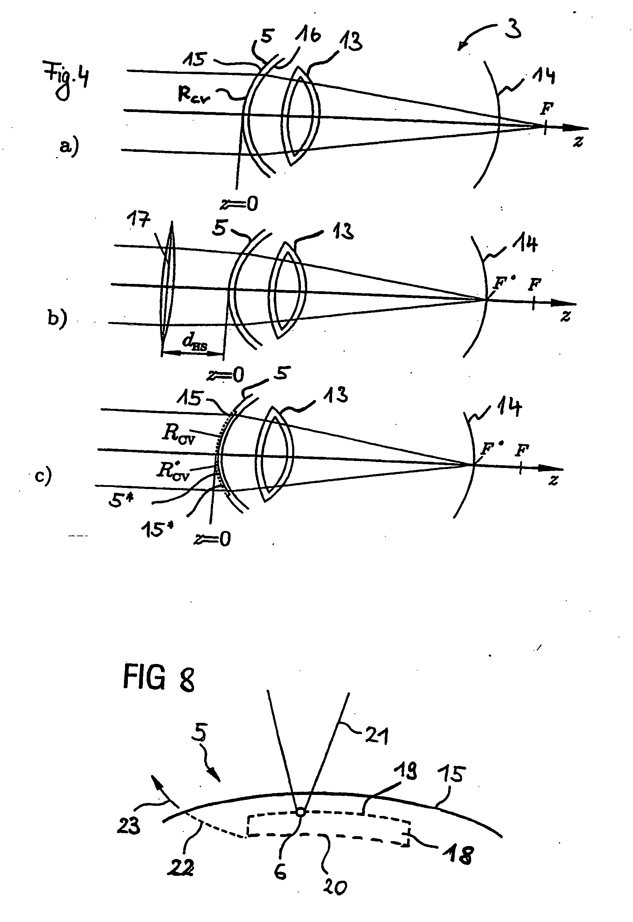

Zuerst gilt es das in der Hornhaut 5 zu isolierend und später zu entfernende Volumen festzulegen. Wie bereits anhand Fig. 1a geschildert bedarf es dazu einer Feststellung des Korrekturbedarfs. Figur 4 zeigt in Teilfiguren a), b) und c) die optischen Verhältnisse am Auge 3 des Patienten 4. Ohne Fehlsichtigkeitskorrektur liegt die in Teilfigur a) gezeigte Situation vor. Die Hornhaut 5 bewirkt zusammen mit der Augenlinse 13 eine Fokussierung eines im Unendlichen liegenden Gegenstandes in einen Fokus F, der auf der z-Achse hinter der Netzbaut 14 liegt. Die abbildende Wirkung rührt dabei zum einen von der bei nichtakkomodiertem Auge entspannten Augenlinse 13 sowie zum anderen von der Augenhornhaut 5 her, die im wesentlichen durch eine Hornhautvorderfläche 15 sowie eine Hornhautrückseite 16 definiert ist und aufgrund ihrer Krümmung ebenfalls eine abbildende Wirkung hat. Die optische Wirkung der Hornhaut 5 ist durch den Krümmungsradius $R_{CV}$ der Hornhautvorderfläche bedingt. Teilfigur a) stellt die Fehlsichtigkeit nur exemplarisch dar, real können die oben erwähnten komplexeren Fehlsichtigkeiten vorliegen. Für sie gilt die nachfolgenden Beschreibung jedoch ebenfalls, allerdings können die angegebenen Gleichungen dann mitunter eine zusätzliche Winkelabhängigkeit beinhalten, auch wenn darauf nicht ausdrücklich hingewiesen wird.

Zur Fehlsichtigkeitskorrektur wird bekannter Weise, wie in Teilfigur b) der Figur 4 dargestellt, eine Vorsatz-Linse 17 in Form einer Brille im Abstand $d_{HS}$ vom Scheitelpunkt der Hornhaut 5 vor das Auge 3 gesetzt. Die Linse 17 der Brille ist in ihrer Brechkraft $B_{BR}$ so angepaßt, daß sie den Fernpunkt des gesamten Systems, d.h. aus Brille und Auge, vom Fokuspunkt F zum korrigierten Fokuspunkt F* verschiebt, der auf der Netzhaut 14 liegt.

Hinsichtlich der in dieser Beschreibung verwendeten Nomenklatur sei angemerkt, daß durch die Anfügung eines Sterns an Größen verdeutlicht wird, daß es sich um Größen handelt, die nach einer Korrektur erhalten werden. Der Fokus F\* ist also derjenige Fokus, der nach der optischen Korrektur vorliegt, die in der Teilfigur b) der Figur 4 durch die Linse 17 der Brille erreicht wird.

Unter der gerechtfertigten Annahme, daß eine Dickenänderung der Hornhaut 5 im wesentlichen den Krümmungsradius der Luft zugewandten Hornhaut-Vorderseite 15 modifiziert, nicht aber den Krümmungsradius der dem Augeninneren zuliegenden Hornhautrückseite 16, wird durch die Volumenentfernung der Krümmungsradius $R_{CV}$ der Hornhautvorderseite 15 modifiziert. Die um das Volumen verminderte Hornhaut 5 hat eine derart geänderte Abbildungswirkung, daß der dann korrigierte Fokus F\* auf der Netzhaut 14 liegt. Nach der Korrektur liegt eine veränderte Hornhautvorderfläche 15\* vor, und es ist eine Fehlsichtigkeitskorrektur auch ohne Brille erreicht.

Zur Bestimmung des Musters der Zielpunkte wird deshalb die zu erreichende Krümmung der modifizierten Hornhautvorderfläche 15\* ermittelt. Dabei ist Ausgangspunkt die Brechkraft der Linse 17 der Brille, da die Ermittlung der entsprechenden Parameter ein Standardverfahren in der Augenoptik ist. Für die Brechkraft $B_{BR}(\varphi)$ der Linse 17 der Brille gilt folgende Formel:

$$(1) \qquad B_{BR}(\varphi) = Sph + Cyl \cdot \sin^2(\varphi - \theta).$$

In dieser Gleichung bezeichnen Sph und Cyl die zu realisierenden Korrekturwerte spärischen bzw. astigmatischen Brechungsfehler und $\theta$ die Lage der Zylinderachse der zylindrischen (astigmatischen) Fehlsichtigkeit, wie sie dem Fachmann in der Optometrie bekannt sind. Der Parameter $\varphi$ schließlich bezieht sich auf ein Zylinderkoordinatensystem des Auges und wird auf das Auge schauend entgegen dem Uhrzeigersinn gezählt, wie es in der Augenoptik üblich ist. Mit dem Wert $B_{BR}$ wird nun die Krümmung der modifizierten Hornhautvorderfläche 15\* wie folgt eingestellt:

$$(2) \qquad R_{CV}{}^* = 1 / ( (1/R_{CV}) + B_{BR} / ( (n_c-1) (1 - d_{HS} \cdot B_{BR}))) + F$$

In Gleichung (2) bezeichnet $n_c$ die Brechkraft des Materials der Hornhaut. Der entsprechende Wert liegt üblicherweise bei 1,376; $d_{HS}$ bezeichnet den Abstand, in dem eine Brille mit der Brechkraft $B_{BR}$ vom Hornhautscheitel liegen muß, um die gewünschte Fehlsichtigkeitskorrektur mittels Brille zu erzeugen; $B_{BR}$ bezeichnet die zuvor erwähnte Brechkraft der Brille gemäß Gleichung (1). Die Angabe für die Brechkraft $B_{BR}$ kann auch Fehlsichtigkeiten erfassen, die über eine normale sphärische oder zylindrische Korrektur hinausgehen. $B_{BR}$ (und damit automatisch auch $R_{CV}{}^*$) haben dann zusätzliche Koordinatenabhängigkeiten.

Der Faktor F drückt die optische Wirkung der Dickenänderung der Hornhaut aus und kann in erster Näherung als konstanter Faktor angesehen werden. Für eine hochgenaue Korrektur kann der Faktor gemäß folgender Gleichung errechnet werden:

$$(3) \qquad F = (1 - 1/n_c) \cdot (d_C{}^* - d_C).$$

$d_C$ bzw. $d_C{}^*$ ist dabei die Hornhautdicke vor bzw. nach der optischen Korrektur. Für eine genaue Bestimmung erfolgt eine Berechnung von $R_{CV}{}^*$ iterativ, indem bei der i- ten Berechnung aus der Differenz $(R_{CV}{}^*- R_{CV})$ auf die Größe $(d_C{}^*- d_C)$ geschlossen wird und das entsprechende daraus erhaltene Ergebnis für die Dickenänderung bei der (i+1)- ten Berechnung angewendet wird. Dies kann man so lange durchführen, bis ein Abbruchkriterium erfüllt wird, beispielsweise wenn die Differenz des Ergebnisses für die Dickenänderung bei zwei aufeinanderfolgenden Iterationsschritten unter einer entsprechend festgelegten Grenze liegt. Diese Grenze kann beispielsweise über eine konstante Differenz festgelegt werden, die einer für die Behandlung angemessene Genauigkeit der Refraktionskorrektur entspricht.

Vernachlässigt man die Dickenänderung der Augenhornhaut, was für ein vereinfachtes Verfahren durchaus zulässig ist, kann F in Gleichung (2) für eine vereinfachte Berechnung auch gleich Null gesetzt, also vernachlässigt und weggelassen werden. Man erhält überraschenderweise folgende einfache Gleichung für die Brechkraft der modifizierten Hornhaut 5\*:

$$B_{CV}^* = B_{CV} + B_{BR} / (1 - B_{BR} \cdot d_{HS})$$

Aus dieser Gleichung ergibt sich für den Fachmann auf einfache Art und Weise mittels der Gleichung $B_{CV}^* = (n-1) / R_{CV}^*$ der Radius $R_{CV}^*$ der Hornhautvorderfläche 15*, der nach der Modifikation vorliegen muß, um die gewünschte Fehlsichtigkeitskorrektur zu erhalten, zu: $R_{CV}^* = 1 / ((1/R_{CV}) + B_{BR} / ((n_c-1) (1 - d_{HS} \cdot B_{BR})))$

Für das Volumen, dessen Entfernung die obige Krümmungsänderung der Hornhautvorderfläche 15 bewirkt, wird nun die das Volumen isolierende Grenzfläche festgelegt. Dabei ist vorzugsweise zu berücksichtigen, daß sich der Durchmesser des zu korrigierenden Bereichs und damit der Durchmesser des zu entnehmenden Volumens möglichst über die Pupillengröße bei dunkelangepaßtem Auge erstrecken sollte.

In einer ersten Variante wird mittels dem Fachmann bekannter numerischer Methoden eine Freifläche definiert werden, die ein Volumen umschreibt, dessen Entfernung die Krümmungsänderung bewirkt. Dazu wird entlang der z-Achse die Dickenänderung ermittelt, die zur gewünschten Krümmungsmodifikation nötig ist. Daraus ergibt sich das Volumen als Funktion von r, φ (in Zylinderkoordinaten) und daraus wiederum dessen Grenzfläche.

Eine einfache analytische Rechnung liefert die folgende zweite Variante, bei der die Grenzfläche des Volumens durch zwei Teilflächen aufgebaut wird, eine zur Hornhautoberfläche 15 hinliegende anteriore Teilfläche und eine gegenüberliegende posteriore Teilfläche. Die entsprechenden Verhältnisse zeigt Figur 5. Das Volumen 18 ist zur Hornhautvorderfläche 15 hin durch eine anteriore Schnittfläche 19 begrenzt, die in konstantem Abstand $d_F$ unter der Hornhautvorderfläche 15 liegt. Diese anteriore Schnittfläche 19 wird in Analogie zur Laserkeratomen auch als Flap-Fläche 19 bezeichnet, da sie dort dazu dient, in Kombination mit einem Öffnungsschnitt zum Rand hin die Augenhornhaut 5 eine Lamelle in Form eines "Flap" von der darunterliegenden Hornhaut 5 abheben zu können. Diese Art der Entnahme des zuvor isolierten Volumens 18 ist natürlich auch hier möglich.

Die anteriore Schnittfläche 19 hat einen Krümmungsverlauf, der um $d_F$ unter der Hornhautvorderfläche 15 liegt. Ist diese sphärisch, ist kann für die Flap-Fläche 19 ein Krümmungsradius angegeben werden, der um $d_F$ geringer ist als der Krümmungsradius $R_{CV}$. Wie später für bevorzugte Varianten beschrieben wird, kann bei der Erzeugung der Schnittfläche 19 durch ein Kontaktglas dafür gesorgt werden, daß die Hornhautvorderfläche 15 zum Zeitpunkt der Schnittflächenerzeugung sphärisch ist, so daß das Muster der Zielpunkte eine sphärische Schnittfläche bewirkt. Die Relaxation des Auges 3 nach Abnahme des Kontaktglases mag dann zwar zu einer nicht-sphärischen Schnittfläche 19 führen, sie hat aber dennoch konstanten Abstand zur Hornhautvorderfläche 15 bzw. 15*. Dies wird später noch erläutert.

Posterior ist das Volumen 18, das aus der Hornhaut 5 entfernt werden soll, durch eine posteriore Schnittfläche 20 begrenzt, die schon grundsätzlich nicht zur Hornhautvorderfläche 15 in konstantem Abstand sein kann. Die posteriore Schnittfläche 20 wird deshalb so ausgebildet sein, daß das Volumen 18 in Form eines Lentikels vorliegt, weshalb die posteriore Schnittfläche 20 auch als Lentikel-Fläche 20 bezeichnet wird. In Figur 5 ist sie exemplarisch als ebenfalls sphärische Fläche mit einem Krümmungsradius $R_L$ eingezeichnet, wobei natürlich das Zentrum dieser Krümmung nicht mit dem Krümmungszentrum der in Figur 5 ebenfalls sphärischen Homhautvorderfläche 15 zusammenfällt.

Figur 6 zeigt die Verhältnisse nach Entfernung des Volumens 18. Der Radius der modifizierten Hornhautvorderfläche 15* beträgt nun $R_{CV}^*$ und kann beispielsweise gemäß den zuvor beschriebenen Gleichungen berechnet werden. Die Dicke $d_L$ des entnommenen Volumens 18 ist dabei maßgeblich für die Radiusänderung, wie Figur 7 verdeutlicht. In dieser Figur sind als weitere Größen noch die Höhe $h_F$ der durch die anteriore Schnittfläche 19 definierten Kugelkappe, die Höhe $h_L$ der durch die posteriore Schnittfläche 20 definierten Kugelkappe sowie die Dicke $d_L$ des zu entfernenden Volumens 18 eingezeichnet.

Die posteriore Schnittfläche 20 legt aufgrund des konstanten Abstandes zwischen Hornhautvorderfläche 15 und anteriorer Schnittfläche 19 den Krümmungsverlauf der Hornhautvorderfläche 15* nach Entfernung des Volumens 18 fest. Somit wird die posteriore Schnittfläche 20 z.B. bei einer zylindrische Parameter berücksichtigenden Fehlsichtigkeitskorrektur einen winkelabhängigen Krümmungsradius haben. Für die in Figur 7 gezeigte Lentikel-Fläche 20 gilt allgemein:

$$R_L(\varphi) = R_{CV}^*(\varphi) - d_F ,$$

bzw. in Zylinderkoordinaten (z, r, φ)

$$z_L(r, \varphi) = R_L(\varphi) - (R_L^2(\varphi) - r^2)^{1/2} + d_L + d_F.$$

Ohne Berücksichtigung eines Astigmatismus entfällt die Abhängigkeit von φ und die Lentikel-Fläche 20 ist sphärisch. Die Lentikel-Fläche 20 besitzt aber, geht man vom Bedarf für eine zylindrische Fehlsichtigkeitskorredtur aus, in der Regel auf verschiedenen Achsen unterschiedliche Krümmungsradien, wobei diese natürlich meist den gleichen Scheitelpunkt haben.

Damit wird weiter automatisch deutlich, daß im Fall einer Zylinderkorrektur die theoretische Schnittlinie zwischen Fläp-Fläche 19 und Lentikel-Fläche 20 nich in einer Ebene, d.h. bei konstanten z-Koordinaten liegt. Der kleinste Krümmungsradius der Lentikel-Fläche 20 liegt bei $φ = θ + π/2$, der größte natürlich auf der Achse θ der zylindrischen Fehlsichtigkeit, d.h. bei $φ = θ$. Bei einer Übersichtigkeitskorrektur fallen anders bei der Darstellung der Figur 7 der Scheitelpunkt von Flap-Fläche 19 und Lentikel-Fläche 20 zusammen und die Lentikel-Fläche 20 ist stärker gekrümmt, als die Flap-Fläche 19. Die Dicke $d_L$ des Lentikels ergibt sich als Randdicke.

Das als Lentikel aufzufassende Volumen 18 hat bei $φ = θ = π/2$ die geringste Randdicke, da sich dort Lentikel-Fläche 20 und Flap-Fläche 19 schneiden. Bei allen anderen Werten für φ ist eine endliche Randdicke gegeben, wenn eine gegebene z-Koordinate als untere Grenze der Lentikel-Fläche 20 angesetzt wird.

Alternativ kann neben der Flap-Fläche 20 und der Lentikel-Fläche 19 zusätzliche Randfläche vorgesehen werden, welche das Volumen 18 im Schnittbereich von Flap-Fläche 20 und der Lentikel-Fläche 19 umrandet bzw diese Flächen dort verbindet, wo sie bei einer gegebenen z-Koordinate nicht zusammenlaufen. Der Schnitt dieser Randfläche wird ebenfalls mit dem gepulsten Laserstrahl ausgefürt. Die Randfläche kann beispielsweise eine zylindrische Form haben, die jedoch auch eine elliptische Form (in der Aufsicht) oder auch eine konische Form (in der Seitenansicht haben kann).

Die in den Figuren gezeigte Ausbildung des Volumens 18 als durch eine anteriore Schnittfläche 19 mit konstantem Abstand zur Homhautvorderfläche 15 sowie eine posteriore Schnittfläche 20 begrenzt, ist nur eine Variante zur Begrenzung des Volumens 18. Sie hat jedoch den Vorteil, daß die optische Korrektur wesentlich nur durch eine Fläche (die Lentikelfläche 20) festgelegt wird, so daß die analytische Beschreibung der anderen Teilfläche der Grenzfläche einfach ist.

Weiter sind optimale Sicherheitsmargen hinsichtlich des Abstandes des Volumens zur Homhautvorderfläche 15 und Hornhautrückfläche 16 geboten. Die Restdicke $d_F$ zwischen anteriorer Schnittfläche 19 und Homhautvorderfläche 15 kann konstant auf einen Wert von beispielsweise 50 bis 200 µm eingestellt werden. Insbesondere kann sie so gewählt sein, daß das schmerzempfindliche Epithel in der Lamelle verbleibt, die durch die Flap-Fläche 19 unter der Hornhautvorderfläche 15 gebildet ist. Auch steht die Ausbildung der sphärischen Flap Fläche 19 in Kontinuität mit bisherigen Keratometerschnitten, was für die Akzeptanz der Methode vorteilhaft ist.

Nach Erzeugen der Schnittflächen 19 und 20 wird dann das derart isolierte Volumen 18 aus der Hornhaut 5 entfernt. Dies ist schematisch in Figur 8 dargestellt, die zudem verdeutlicht, daß die Schnittflächen 19 und 20 durch Einwirkung des in einem Fokuskegel 21 einfallenden Behandlungslaserstrahls erzeugt werden, beispielsweise durch Aneinanderreihung von Plasmablasen, so daß in einer bevorzugten Ausführungsform die Flap-Schnittfläche 19 und die Lentikel-Schnittfläche 20 durch geeignete dreidimensionale Verstellung der Fokuslage der gepulsten Laserstrahlung 2 erzeugt werden.

Alternativ kann in einer vereinfachten Ausführungsform aber auch lediglich die Flap-Fläche 19 durch Zielpunkte, die die gekrümmte Schnittfläche 19 in konstantem Abstand zu Hornhautvorderfläche 15 definieren mittels gepulster Laserstrahlung gebildet werden und die Entfernung des Volumens 18 erfolgt durch Laserablation, beispielsweise durch Verwendung eines Excimers-Laserstrahls. Hierzu kann die Lentikel-Fläche 20 als Grenzfläche des Abtrages definiert werden, auch wenn das nicht zwingend erforderlich ist. Das Behandlungsgerät 1 arbeitet da wie ein bekanntes Laserkeratom, allerdings wird die Schnittfläche 19 an gekrümmter Hornhaut erzeugt. Die vorangehend bzw. nachfolgend beschriebenen Merkmale sind auch in solchen Varianten möglich, insbesondere was die Bestimmung der Begrenzungsfläche, deren geometrische Definition und die Ermittlung von Steuerparametern angeht.

Erzeugt man sowohl die Lentikel-Fläche 20 als auch die Flap-Fläche 19 mittels gepulster Laserstrahlung, ist es zweckmäßig, die Lentikel-Fläche 20 vor der Flap-Fläche 19 auszubilden, da das optische Ergebnis bei der Lentikel-Fläche 20 besser (wenn nicht überhaupt erst zu erreichen) ist, wenn oberhalb der Lentikel-Fläche 20 noch keine Veränderung der Hornhaut 5 eintrat.

Das Entfernen des durch die gepulste Laserstrahlung isolierten Volumens 18 kann, wie in Figur 8 angedeutet, durch einen Randschnitt 22 erreicht werden, der es erlaubt, das Volumen 18 in Richtung eines in Figur 8 eingezeichneten Pfeiles 23 herauszuziehen. Alternativ kann der Randschnitt 22 aber so ausgebildet werden, daß er die anteriore Schnittfläche 19, d. h. die Flap-Fläche 19, in Form eines Ringes mit der Homhautvorderfläche 15 verbindet, wobei der Randschnitt allerdings nicht vollständig um einen Winkel von 360° umläuft. Die derart isolierte Lamelle bleibt in einem schmale Bereich mit dem übrigen Gewebe der Hornhaut 5 in Verbindung. Diese Verbindungsbrücke dient dann als Gelenk, um die ansonsten isolierte Lamelle von der Hornhaut 5 abzuklappen und das dadurch zugängige, bereits isolierte Volumen 18 vom Rest der Augenhornhaut 5 abnehmen zu können. Die Lage der Verbindungsbrücke ist bei Erzeugung der Steuerdaten bzw. der Zielpunkte vorgebbar. Das beschriebene Vorgehen bzw. Gerät realisiert also unter diesem Gesichtspunkt die Isolierung des Volumens 19 innerhalb der Hornhaut 5 und das Erzeugen einer mit der restlichen Augenhornhaut über eine Gewebebrücke verbundenen Lamelle als Deckel über dem Volumen. Der Deckel kann abgeklappt und das Volumen 18 entnommen werden.

Für die Erzeugung der Schnittflächen 19 und 20 können die Zielpunkte nun auf verschiedenste Art und Weise angeordnet werden. Im Stand der Technik ist beispielsweise in der WO 2005/011546 zur Erzeugung von Schnittflächen in der Augenhornhaut beschrieben, daß spezielle Spiralen eingesetzt werden können, die beispielsweise um eine im wesentlichen senkrecht zur optischen Achse (z-Achse) liegende Hauptachse in Art einer Schraubenlinie verlaufen. Auch ist die Verwendung eines Scanmusters bekannt, das die Zielpunkte zeilenweise anordnet (vgl. WO 2005/011545). Diese Möglichkeiten können selbstverständlich zur Erzeugung der oben definierten Schnittflächen verwendet und mit den nachfolgend erläuterten Transformationen kombiniert werden.

Die Verstellung der Lage des Fokus in der Augenhornhaut erfolgt mittels der in Figur 3 schematisch dargestellten dreidimensionalen Ablenkeinrichtung, die zur Verstellung des Fokus in z-Richtung die Verschiebung von Linsen oder anderer optisch wirksamer Elemente einsetzt. Nun ist die Verstellung von Linsen o.ä. in der Regel nicht so schnell möglich, wie die Verschwenkung von Spiegeln, wie sie in der Regel im xy-Scanner Einsatz finden. Meist ist deshalb die Versteligeschwindigkeit des z-Scanners begrenzend für die Geschwindigkeit, mit der die Schnittflächen in der Augenhornhaut erzeugt werden können. Zur möglichst schnellen Erzeugung der Schnittflächen 18 und 19 wird deshalb in einer bevorzugten Ausführungsform der Fokus jeweils entlang einer spiralförmigen Bahn geführt, wobei je eine Spirale in der räumlich gekrümmten Schnittfläche liegt. Während des Schreibens der Spirale wird also der z-Scanner so verstellt, daß die Arme der Spirale der räumlich gekrümmten Schnittfläche folgen.

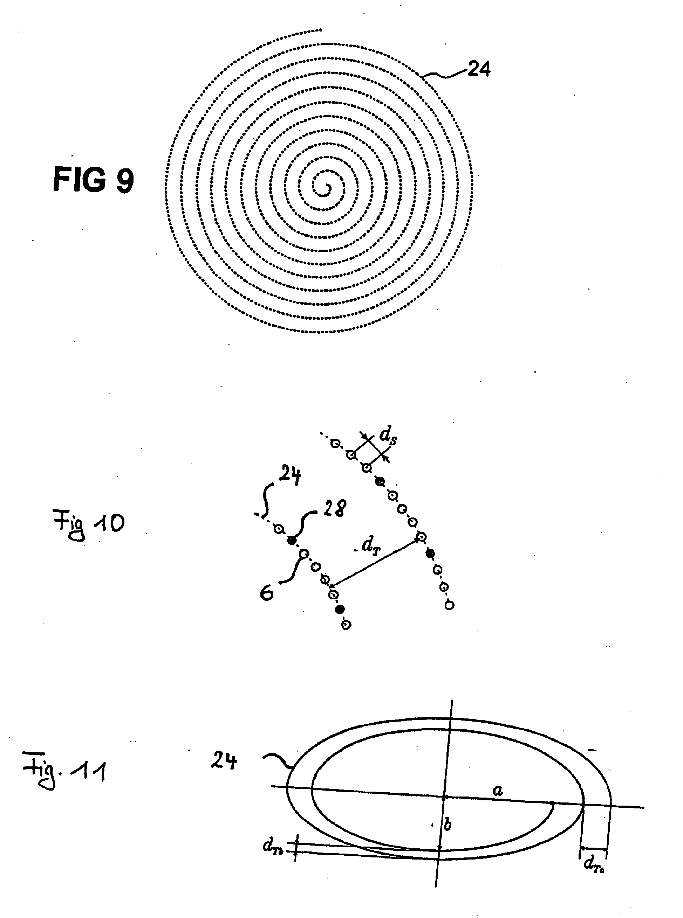

Figur 9 zeigt exemplarisch eine Bahnkurve 24 als Spirale, die in der gezeigten Darstellung als Kreisspirale ausgebildet ist. Der Radius der dargestellten ebenen Spirale nimmt in Kreiskoordinaten mit steigendem Drehwinkel $\varphi$ zu, so daß gilt:

$$(4) \qquad r(\varphi) = \varphi \cdot d_T / (2\pi)$$

In dieser Gleichung bezeichnet $d_T$ den Abstand der Spiralarme; er ist in Figur 10 dargestellt, die einen vergrößten Ausschnitt der Figur 9 zeigt. Der Abstand der einzelnen Spots 6, auf die gepulste Laserstrahlung fokussiert wird und an denen durch einen Laserpuls beispielsweise eine Plasmablase erzeugt wird, ist in der Spirale konstant gleich $d_s$, so daß für den Winkelabstand $\Delta\varphi$ der einzelnen Spots 6, an denen ein Laserpuls in das Gewebe eingebracht wird, gilt:

$$\Delta\varphi = d_S / r$$

Da, wie bereits erwähnt, die Lentikel- Fläche 20 in der Regel nichtsphärisch ist, ist die Bahnkurve 24, entlang der der Laserfokus verstellt wird, eine elliptische Spirale, für die natürlich kein konstanter- Abstand der Spiralarme mehr gegeben ist. Entlang- der Hauptachsen a und b kann aber ein jeweiliger Bahnabstand $d_{Tb}$ sowie $d_{Ta}$ definiert werden, wie Figur 11 zeigt.

In Figur 10 sind die Spots 6 dargestellt, um die Lage des Fokus für die einzelnen Laserpulse erkennen zu lassen. Tatsächlich weiten sich die Plasmablasen natürlich nach Einbringung des jeweiligen Laserpulses so weit auf, daß die Schnittfläche erzeugt wird, und die Bahnkurve 24 ist in der Schnittfläche dann nicht mehr zu erkennen.

Zur Vorbereitung des chirurgischen Verfahrens muß nach der Definition der Schnittflächen 19 und 20 nun die Definition der Bahnkurven 24 erfolgen, mit denen die Schnittflächen erzeugt werden.

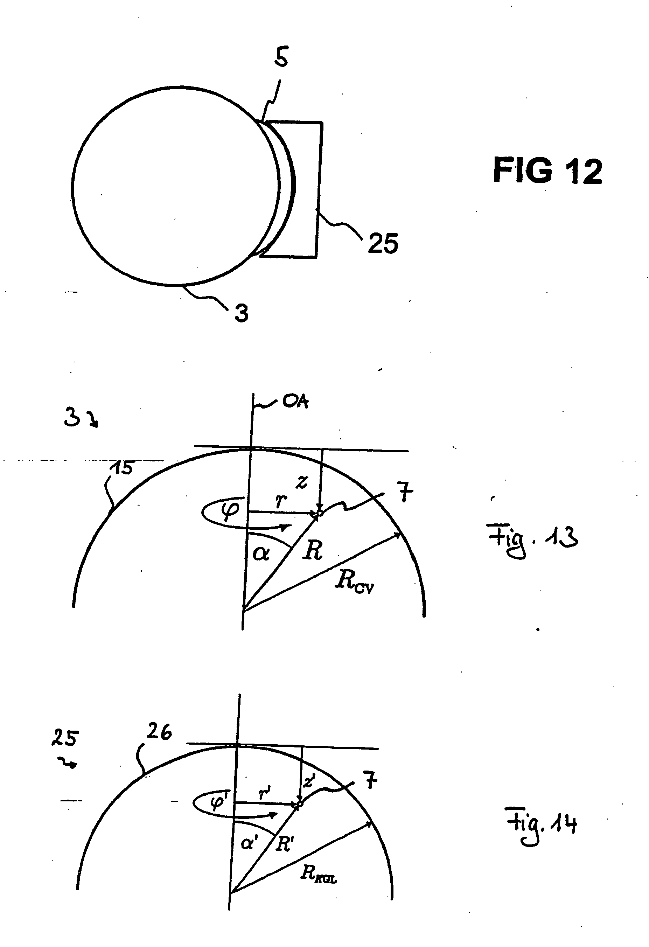

Bei der Bestimmung der Bahnkurven 24 ist natürlich zu berücksichtigen, daß letztendlich das Volumen 18 im Auge im Normalzustand definiert sein soll. Die Schnittflächen 19 und 20, wie sie bislang erläutert wurden, betreffen das natürliche Auge. Es ist nun aber zu berücksichtigen, daß das Behandlungsgerät 1 aus Gründen der Fixierung des Auges mit einem Kontaktglas 25 arbeitet, das wie in Figur 12 gezeigt ist, auf die Homhautvorderfläche 15 der Augenhornhaut 5 aufgesetzt wird. Das Kontaktglas 25, das bereits Gegenstand mehrerer Patentpublikationen ist (exemplarisch sei beispielsweise auf die WO 2005/048895 A verwiesen), ist für die hier vorliegende Beschreibung des Behandlungsgerätes 1 bzw. der damit in Zusammenhang stehenden Verfahren zur Vorbereitung und/oder Durchführung des chirurgischen Eingriffes allerdings nur insoweit von Interesse, als es der Hornhautvorderfläche 15 zum einen eine definierte Krümmung verleiht und zum anderen die Augenhornhaut 5 gegenüber dem Behandlungsgerät 1 räumlich in einer vordefinierten Lage hält. Hinsichtlich der sphärischen Krümmung der Kontaktfläche des Kontaktglases 25 unterscheidet sich der hier beschriebene Ansatz jedoch deutlich von dem Ansatz, wie er beispielsweise in der WO 2003/002008 A beschrieben ist, der ein planes Kontaktglas verwendet, welches die Augenhornhaut flachdrückt.

Wird das Auge an das Kontaktglas 25 mit sphärischer Kontaktfläche angepreßt, kommt es zu einer räumlichen Deformation des Auges. Da die Komea regelmäßig nur tangential kompressibel ist, also bei einem solchen Anpressen ihre Dicke nicht ändert, entspricht das Anpressen einer Transformation vom Koordinatensystem des Auges, wie es in Figur 13 dargestellt ist, in das Koordinatensystem des Kontaktglases, das in Figur 14 gezeigt ist. Dieser Zusammenhang ist dem Fachmann aus der WO 2005/011547 A1 bekannt, deren Offenbarungsgehalt diesbezüglich vollumfänglich eingebunden sein soll. In den Figuren 13 und 14 bezeichnen mit einem Apostroph versehene Koordinaten die Koordinaten des auf das Kontaktglas 25 bzw. dessen dem Auge zugewandte Kontaktglasunterseite 26 bezogene Größen.

Das Kontaktglas hat aber noch einen weiteren Vorteil. Durch das Anpressen an die sphärische Kontaktglasunterseite 26 ist automatisch auch die Hornhautvorderfläche 15 sphärisch. Die in konstantem Abstand unter der Homhautvorderfläche 15 liegende anteriore Schnittfläche 19 ist damit bei angepreßtem Kontaktglas ebenfalls sphärisch, was zu erheblich vereinfachter Ansteuerung führt. Es ist deshalb völlig unabhängig von anderen Merkmalen bevorzugt, ein Kontaktglas 25 mit sphärischer Kontaktglasunterseite 26 zu verwenden und das Volumen durch eine anteriore Schnittfläche 19 sowie eine posteriore Schnittfläche zu begrenzen, wobei für die anteriore Schnittfläche. Zielpunkte vorgegeben sind/werden, die diese Schnittfläche als sphärische Fläche in konstantem Abstand $d_F$ unter der Hornhautvorderfläche 15 ausbilden. Für die posteriore Schnittfläche sind/werden Zielpunkte vorgegeben, die einen Krümmungsverlauf definieren, welcher bei relaxiertem Auge, also nach Abnehmen des Kontaktglases, bis auf den Abstand $d_F$ zur Homhautvorderfläche dem zur Fehlsichtigkeitskorrektur gewünschten entsprechen. Analoges gilt für das Verfahren zur Definition der Zielpunkte bzw. das Operationsverfahren.

Die Darstellungen in den Figuren 13 und 14 zeigen die Koordinatentransformation, die am Auge durch das Aufsetzten bzw. Abnehmen des Kontaktglases auftritt. Sie enthalten sowohl Kugelkoordinaten ($R$, $\alpha$, $\varphi$) bezogen auf den Ursprung der gekrümmten Fläche (Homhautvorderfläche 15 bzw. Kontaktglasunterseite 26) als auch Zylinderkoordinaten ($r$, $z$, $\varphi$) bezogen auf den durch den Durchtrittspunkt der optischen Achse OA definierten Scheitelpunkt der Homhautvorderfläche 15 bzw. der Kontaktglasunterseite 26.

Bei der Koordinatentransformation vom auf das Auge bezogenen Koordinatensystem, wie es in Figur 13 dargestellt ist, in das auf das Kontaktglas bezogene System gemäß Figur 14 bleiben die Bogenlänge, d.h. $\alpha \cdot R$, die radiale Tiefe ($R_{CV}$ - $R$) sowie der Winkel $\varphi$ erhalten. Die Transformation der für das natürliche Auge, d.h. im Koordinatensystem der Figur 13, zugrundegelegten Formen der Schnittflächen 19 und 20 ist somit ein wichtiger Schritt bei der Berechnung der Ansteuergrößen für die dreidimensionale Fokusverstelleinrichtung. Sie verläuft grundsätzlich anders als bei einem ebenen Kontaktglas, in dem z.B. die Flap-Fläche 19 zu einer Ebene entartet. Im Wesentlichen ist nur die Form für die Schnittfläche 20 zu transformieren, da die Schnittfläche 19 lediglich in konstantem Abstand $d_F$ zur Homhautvorderfläche 15 auszubilden ist. Die Schnittfläche 19 ist also im transformierten System eine Sphäre mit einem gegenüber der Kontaktglasunterseite um $d_F$ reduzierten Krümmungsradius $R_F$.

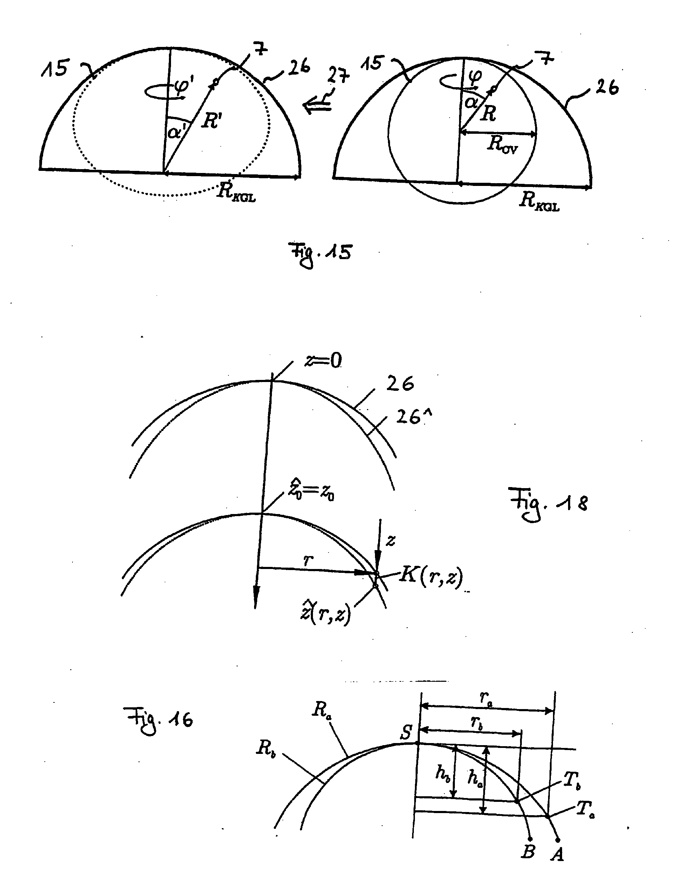

Das Anpressen der Hornhaut 5 des Auges 3 an die sphärisch gekrümmte Kontaktglasunterseite 26 ist in Figur 15 veranschaulicht. Dort zeigt die rechte Darstellung schematisch den Zustand, wenn die Kontaktglasunterseite 26 nur am Scheitelpunkt in Kontakt mit der Hornhautvorderfläche 15 ist. Zur verdeutlichung der geometrischen Beziehungen ist die Homhautvorderfläche 15 schematisch in Figur 15 als Kreis eingezeichnet, obschon natürlich die sphärische Krümmung nur in einem kleineren Kreisabschnitt vorliegt. Das Anpressen des Kontaktglases 25 auf die Hornhaut 5 bewirkt den durch Pfeil 27 symbolisierten Übergang zum Zustand der linken Seite der Figur 15. Das Abnehmen des Kontaktglases 25 bewirkt eine Relaxation des Auges 3 entgegen der Richtung des Pfeiles 27.

Aufgrund der geschilderten Rahmenbedingungen transformieren sich für jeden Punkt in der Augenhornhaut 5 die Koordinaten von dem in Figur 13 dargestellten System in das System der Figur 14. Dies wird nun bei der Wahl der Ansteuerwerte für die Fokusverstellung dahingehend zugrundegelegt, daß die Schnittflächen 19 und 20 im transformierten Kontaktglassystem zu beschreiben sind, da sie nur dann nach Abnehmen des Kontaktglases 26, d.h. nach RückTransformation in das natürliche Koordinatensystem des Auges, die gewünschten Formen haben. Da das Anlegen der Hornhautvorderfläche 15 in der Regel durch Ansaugen mittels Unterdruck bewirkt wird, wird die geschilderte Transformation nachfolgend auch als Ansaugtransformation bezeichnet.

Zum Schneiden der Flap-Fläche 19, die wie erwähnt sphärisch ist, wird nun folgende Geschwindigkeit der Verstellung des z-Scanners, d.h. folgende Vorschubgeschwindigkeit in z-Richtung eingestellt:

$$(5) \qquad v_Z(t) = d_S \cdot f_L \cdot d_T / \left(2\pi \cdot (R_F^2 - t \cdot d_S \cdot f_L \cdot d_T / \pi)\right)^{\frac{1}{2}},$$

wobei $f_L$ die Frequenz der Laserpulse der Laserstrahlung 2 ist. Gleichung (5) setzt voraus, daß die z-Geschwindigkeit $v_Z$ frei eingestellt und kontinuierlich verändert werden kann.

Möchte man eine Sphäre mit einer Geschwindigkeit $v_Z$ schreiben, die aus einer Gruppe diskreter Geschwindigkeiten gewählt ist, was in der Regel dann der Fall ist, wenn der z-Scanner mittels eines Schrittmotors angetrieben ist, erhält man als Zeitabhängigkeit der Radialfunktion $r(t)$:

$$(6) \qquad r(t) = [d_S \cdot f_L \cdot d_T \cdot t/\pi - (d_S \cdot f_L \cdot d_T \cdot t)^2/(2\pi \cdot R_F)^2]^{1/2}$$

sowie für die Winkelfunktion

$$(7) \qquad \varphi(t) = [4\pi \cdot d_S \cdot f_L \cdot t/d_T - (d_S \cdot f_L \cdot t)^2/R^2]^{1/2}.$$

Die $t^2$-Terme unter der Wurzel der Radial- wie der Winkelfunktion zeigen, daß keine ideale archimedrische Spirale mehr geschrieben wird, die Bahn- und Spotblasen-Abstände variieren also zugunsten der nur in Stufen veränderlichen z-Geschwindigkeit.

Wünscht man bei der Fokusverstellung einen konstanten z-Vorschub, ergibt sich nicht, wie mit der Geschwindigkeit gemäß Gleichung (4) eine Sphäre, sondern ein Paraboloid, und es gilt:

$$(8) \qquad z(r) = [v_Z/(d_S \cdot d_T)][r^2 \cdot \pi/f_L]$$

Erwähnterweise kann in manchen Behandlungsgeräten 1 die Geschwindigkeit, mit der der z-Scanner den Fokus in z-Richtung verschiebt, nur innerhalb eines Satzes diskreter Geschwindigkeiten verstellt werden. Möchte man dann eine bestimmte Parabel mit einer gegebenen Geschwindigkeit $v_Z$ beschreiben, muß das Produkt $d_S \cdot d_T$ entsprechend gewählt werden, so daß die erste eckige Klammer der Gleichung (8) den gewünschten Wert einnimmt. Der Abstand der Bahnen, definiert durch $d_T$, sowie der Spotabstand entlang der Bahnbeschrieben durch $d_S$, sind also geeignet zu variieren, um eine bestimmte Parabel mit gegebenen $v_Z$ zu schreiben.

Jede der Gleichungen (5), (6)/(7) und (8) kann bei der Ermittlung der Zielpunkte und damit de Ansteuerung der Fokusverstellung verwendet werden, wobei dann natürlich die entsprechende Spiralform/Flächenform zugrundezulegen ist. Wenn nachfolgend davon gesprochen wird, daß die Gleichungen bei der Ansteuerung verwendet werden, ist darunter insbesondere zu verstehen, daß mittels der Gleichungen die Zielpunkte ermittelt werden, die kann z.B. durch Auswerten der Funktionsgleichungen zu äquidistanten Zeitpunkten geschehen. Die Geschwindigkeitsgleichungen werden in einer Variante dazu verwendet, sicherzustellen, daß die ermittelten Zielpunkte keine Verstellgeschwindigkeiten bedingen, die von der Fokusverstelleinrichtung gar nicht realisierbar sind.

Für die eingangs erwähnten und wie beschrieben ermittelten Formen der Flächen 19 und 20 wird nun eine Spirale in die jeweilige Fläche gelegt. Die Spirale wird durch eine Ansteuerung der beschriebenen Art geschrieben. Die Berechnung der z-Geschwindigkeit sowie der r- und $\varphi$-Geschwindigkeit berücksichtigt dabei, welche Flächenform die Fläche 19 bzw. 20 hat.

Die Lentikel-Fläche 20 ist sphärisch, wenn keine Zylinderkorrektur vorgenommen werden soll. Man verwendet deshalb die Ansteuerung gemäß Gleichungen (4)/(5) oder (6)/(7) um diese Sphäre zu erzeugen. Allerdings kann eine Sphäre bekanntermaßen auch durch ein Paraboloid approximiert werden. Es ist deshalb in einer Variante vorgesehen, die eine Sphäre auf dem Fachmann bekannter Weise durch ein Paraboloid anzunähern und die Ansteuerung gemäß Gleichung (8) vorzunehmen.

Durch die Ansaugtransformation gemäß Figur 15 verändert sich die Geometrie der Lentikel-Fläche 20. Die Lentiköl-Fläche 20 muß im Koordinatensystem des Kontaktglases 25 den Krümmungsverlauf der korrigierten Homhautvorderfläche 15* aufweisen. Sie kann nicht auf einen Krümmungsmittelpunkt bezogen werden, der mit dem Krümmungsmittelpunkt des Kontaktglases zusammenfällt. Die gemäß Gleichung (2) definierte Krümmung wird also bezüglich der Ansaugtransformation umgerechnet.

Der in Gleichung (2) definierte Krümmungsradius ist natürlich eine Funktion von $\varphi$. Wie bereits erwähnt und in der Augenoptik üblich, können zwei Krümmungsradien $r_a$ bzw. $r_b$ angegeben werden: einer auf der Achse $\theta$ der zylindrischen Fehlsichtigkeit und einer für eine Achse rechtwinklig dazu. Rechentechnisch ist es besonders günstig, die sich somit im allgemeinen Fall einstellende toroidale Krümmung durch eine Parabel zu approximleren, so daß die Lentikel-Fläche 20 durch ein Paraboloid angenähert wird. Dies geschieht vorzugsweise vor der Anpreßtransformation kann aber auch danach durchgeführt werden.

Die Annäherung erfolgt dadurch, daß man für die zwei Krümmungsradien jeweils eine Parabel sucht, die sowohl durch den Scheitelpunkt der Lentikel-Fläche 20 als auch durch einen möglichst am Rand gelegenen Punkt läuft. Die entsprechenden Verhältnisse im Koordinatensystem des Auges zeigt Figur 16. Diese Figur zeigt die sphärische Lentikel-Fläche 20 vor der Anpreßtransformation. In der Figur sind die Schnitte durch die im verallgemeinerten Fall toroidale Lentikel-Fläche 20 entlang der zwei Halbachsen a und b übereinandergelegt. Die entsprechenden Kurven sind mit A bzw. B bezeichnet und sind kreisförmig mit einem Krümmungsradius $r_a$ bzw. $r_b$. Auf jeder Kurve ist ein Randpunkt T in Zylinderkoordinaten durch den entsprechenden Radius r sowie die Höhe h beschrieben, wobei diese Parameter auf den Scheitelpunkt S bezogen sind, der für die zwei Halbachsenschnitte identisch ist. Der Punkt $T_a$ ist also durch den Radius $r_a$ sowie die Höhe $h_a$ gekennzeichnet. Analoges gilt für $T_b$.

Es wird nun eine Parabel gesucht, für die gilt $h = k \cdot r^2$. Die dadurch erhaltenen Parabelparameter $h_a$ für die Parabel entlang der großen Halbachse a sowie $k_b$ für die Parabel entlang der kleinen Halbachse b definieren das Paraboloid, das dann unter Verstellung des Fokuspunktes in z-Richtung geschrieben wird, beispielsweise mittels eines konstanten z-Vorschubes (vgl. Gleichung (7)) bzw. die mit einer Auswahl der z-Geschwindigkeit aus einem Satz diskreter Geschwindigkeiten gewählt wird (Modifikation zur Gleichung (7)). Die in Figur 16 dargestellten Schnitte der toriodalen Lentikel-Fläche 20 entlang der kleinen Halbachse b sowie der großen Halbachse a beziehen sich auf die Darstellung im Koordinatensystem des Auges.

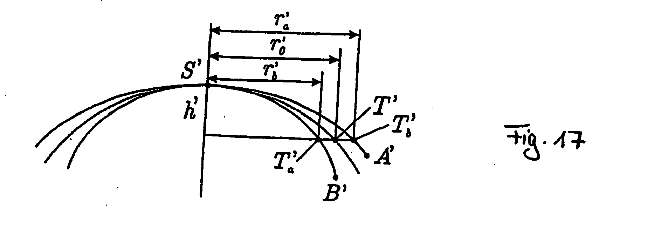

Wenn die Approximation durch Parabelgleichungen nach der Anpreßtransformation durchgeführt wurde, treten dort natürlich die transformierten Werte auf. Man kann die explizire Berechnung von transformierten Werten an dieser Stelle vermeiden, wenn die Approximation zuerst erfolgt und die dabei gefundenen Parabelparameter der Anpreßtransformation in das Koordinatensystem des Kontaktglases unterworfen werden, wonach dann die in Figur 17 dargestellten Verhältnisse vorliegen.

Die Spezifikation der Parabelparameter lautet im Koordinatensystem des Auges gemäß Figur 16 wie folgt:

$$(9) \qquad k_a = ( z(T_a) - z(S) ) / r(T_a)^2,$$

$$(10) \qquad k_b = ( z(T_b) - z(S) ) / r(T_b)^2.$$

In den Gleichungen (9) und (10) bezeichnet z(S) die z-Koordinate des Punktes S. Legt man den Koordinatensystemursprung, wie in den bisherigen Figuren, in den Scheitelpunkt, ist die z-Koordinate Null. Die Koordinate $z(T_a)$ bzw. $z(T_b)$ sowie $r(T_a)$ bzw. $r(T_b)$ sind die z- bzw. r-Koordinaten des entsprechenden Punktes $T_a$ bzw. $T_b$ im zylindrischen Koordinatensystem.

Werden die Parabelparameter $k_a$ bzw. $k_b$ nicht im Koordinatensystem des Auges gemäß Figur 16, sondern im Koordinatensystem des Kontaktglases gemäß Figur 17 benötigt, treten anstelle der Punkte S, $T_a$ und $T_a$ dann die in Figur 17 eingezeichneten transformierten Punkte S', $T_a$', $T_b$'.

Um die Lentikel-Fläche 20 nun im angepreßten Auge 3 durch eine (dann in der Regel elliptische) Spirale mit den Hauptachsen $r_a$' und $r_b$' darzustellen, wird die Spirale aus einer Kreisspirale mit Radius $r_o$' durch Streckung in Richtung $\varphi = \theta$ und Stauchung in Richtung $\varphi = \theta + \pi/2$ konstruiert. Durch die gleichzeitige Stauchung und Streckung bleibt der mittlere Bahn- bzw. Spotabstand erhalten. Würde man nur in eine Richtung stauchen oder strecken, würde der mittlere Abstand verändert.

Die eigentlichen Radien lassen sich aus dem Radius $r_0$ der Kreisspirale, der in Figur 17 gestrichelt eingezeichnet ist, mit Hilfe der Elliptizität wie folgt berechnet:

$$e' = r_a'/r_b' = (k_B'/k_a')^{1/2}.$$

Die Elliptizität e' ist dabei die Elliptizität der transformierten toriodalen Lentikel- Fläche 20. Die Parameter $k_b$' sowie $k_a$' sind durch die Gleichungen (11) und (12) , jeweils für die transformierten Punkte S', $T_a$' und $T_b$' gegeben. Die Parabelparameter ergeben sich daraus, daß hier die Kreisspirale mit dem Radius $r_0$, aus dem die Lentikel- Fläche 20 konstruiert ist, ein arithmetisches oder geometrisches Mittel der Krümmungen der großen bzw. kleinen Halbachse des Paraboloid sein soll. Wegen $r_0' = (r_a' \cdot r_b')^{1/2}$ erhält man für den Parabel parameter $k = (k_a \cdot k_b)^{1/2}$ sowie die Hauptachsen der Ellipse: $r_a' = r_0' \cdot (e')^{1/2}$ und $r_b' = r_0' \cdot (e')^{-1/2}$.

An dieser Stelle der Ermittlung der Zielpunkte liegen nun zwei Bahnkurven 24 vor, die durch Funktionsgleichungen beschrieben sind. Das Muster der Zielpunkte wird durch Auswertung der Funktionsgleichungen ermittelt.

Allerdings bleibt noch zu berücksichtigen, daß die Fokussierung des Laserstrahls in den Fokus 7 einem Fokuslagenfehler unterliegt. Dieser Fokuslagenfehler ist Eigenschaft des optischen Systems, d. h. beruht auf der verwendeten optischen Realisierung. Er ist im wesentlichen durch das optische Design bestimmt. Aufgrund endlicher Fertigungsgenauigkeiten im Rahmen der erlaubten Toleranz ist der Fokuslagenfehler darüber hinaus geräteindividuell. Er wird deshalb zweckmäßigerweise für jedes Gerät eigenständig bestimmt.

Die Berücksichtigung des Fokuslagenfehlers erfolgt durch eine in der Regel nichtlineare Transformation (nachfolgend auch als NL-Transformation bezeichnet). Es ist deshalb nicht möglich, die NL-Transformation durch Modifikation der Bahnkurvenparameter auszuführen. In einer bevorzugten Ausführungsform wird der Fokuslagenfehler durch eine Korrekturtabelle oder eine Korrekturfunktion ausgedrückt. Sie stammt aus einer Vermessung der Optik des Behandlungsgerätes 1. Die Vermessung kann gerätetypbezogen oder geräteindividuell geschehen. Die Korrekturfunktion kann aus einer Interpolation der Meßresultate z. B. mittels Polynomen oder Splines gewonnen sein. Meist ist der Fokuslagenfehler rotationssymmetrisch bezogen auf die optische Achse. Er hängt dann in Zylinderkoordinaten nur von r und z ab.

Die zuvor mittels der Bahnkurven errechneten Punkte werden in der NL-Transformation so vorverzerrt, daß sie nach der Einbringung der Laserspots mit dem optischen System, das den Fokuslagenfehler aufweist, genau an der gewünschten Stelle liegen. Die Anwendung der vorverzerrten Koordinaten kompensiert also den im optischen System auftretende Fokuslagenfehler.

Die NL-Transformation geht von der Überlegung aus, daß man zu jedem Punkt mit den Koordinaten (z, r) eine um $z_0$ verschobene Kontaktglassphäre findet, auf der dieser Punkt liegt. Der Scheitelpunkt dieser Sphäre ist dann gerade bei $z_0(z, r) = z - z_{KGL}(r)$. Die Wirkung der Vorverzerrung zur Kompensation des Fokuslagenfehlers ist in Figur 18 veranschaulicht. Sie zeigt die Kontaktglasunterseite 26, die im vorliegenden Beispiel sphärisch ist, aber auch eine andere Form haben kann. Aufgrund der Vorverzerrung wird sie zu einer transformierten Kontaktglassphäre 26^. Die die Vorverzerrung des Fokuslagenfehlers berücksichtigenden Parameter sind in Figur 18 durch ein angefügtes Dach "^" symbolisiert. Für einen transformierten Achsenpunkt in der Komea gilt dann $z_0{}^\wedge = z_0$. Dies trägt der Tatsache Rechnung, daß der Fokuslagenfehler zwar meist rotationssymmetrisch ist, allerdings auch eine Verschiebung in z-Richtung zur Folge hat.

Zur Vorverzerrung werden die berechneten Bahnkurven in individuelle Zielpunkt-Koordinaten für die Spots umgesetzt, welche dann in der in Figur 18 durch K(r, z) symbolisierten Korrekturtransformation, also der NL-Transformation, verschoben werden. Ist der Fokuslagenfehler als Funktion K angeben, muß die Koordinate eines jeden Zielpunktes lediglich mit der Funktion ausgewertet werden, um die Verschiebung bzw. die transformierte Koordinate zu erhalten.

Im Ergebnis liegt dann für die Flächen 19 und 20 jeweils ein Satz Zielpunkt-Koordinaten vor, entlang der der Fokus geführt wird. Durch die NL-Transformation und die Anpreßtransformation liegen die Koordinaten bezogen auf das natürliche, also freie Auge genau in den gewünschten anterioren und posterioren Schnittflächen 19, 20.

Die so erhaltenen Koordinaten für die Zielpunkte müssen noch in Ansteuersignale für die dreidimensionale Ablenkeinheit, z.B. die xy-Scanner sowie den z-Scanner umgesetzt werden. Hierzu wird ein entsprechender funktioneller Zusammenhang oder ein entsprechendes Kennfeld verwendet, daß für die Scanner bekannt ist und gegebenenfalls vorab ermittelt wurde.

Insbesondere für die xy-Scanner, die im Ausführungsbeispiel als Galvanometerspiegel realisiert sind, wurde zuvor die Response-Funktion bestimmt. Ein Beaufschlagen der Galvanometerspiegel mit einem Frequenz-Sweep sowie Messen der tatsächlichen Galvanometerbewegung liefern eine Amplituden- und Phasen-Antwortfunktion. Diese werden bei der Bestimmung der Ansteuersignale berücksichtigt.

Weiter wird zur vereinfachten Ansteuerung nicht für jeden Punkt dem Scanner ein Signal für das anzufahrende Ziel vorgegeben. Statt dessen bewirkt das Steuergerät 12 eine Vorgabe von Stützstellen, die die Bahn des Scanners charakterisieren. Die Punktezahl ist dadurch deutlich reduziert. Dies kann schon nutzbringend bei der NL-Transformation ausgenutzt werden, indem nur für die Bahnkurven nur diejenigen Zielpunkte der Transformation unterworfen werden, die Stützstellen bei der Ansteuerung sein sollen. Die Auswertung der Funktionsgleichungen erfolgt in einer Ausführungsform also entsprechend einem auf einen zeitlichen Abstands der größer ist, als der Zeitabstand der Laserpulse.

Es wird somit also vor der NL-Transformation eine Filterung der Bahnkurvenpunkte vorgenommen, die die erwähnten Stützstellen, d.h. Punkte in einer Frequenz der Scanneransteuerung zur Transformation vorsieht. Äquivalent mit einer solchen Filterung ist eine Auswertung der funktionsmäßig beschriebenen Bahnkurven an entsprechend der Scannersteuerung beabstandeten Stützstellen. Hier tritt ein weiterer Vorteil des hier geschilderten funktionsbasierten Ansatzes zu Tage: Die Entscheidung, welche Punkte Zielpunkte bei der Ansteuerung der Fokusverstelleinrichtung sind, muß erst vor der NL-Transformation getroffen werden. Zuvor müssen lediglich die Bahnparameter geeignet umgerechnet werden. Auch liegen erst dann Datensätze mit einer Vielzahl an Punkten vor.

Die Stützstellen definieren somit Zielpunkte, die nur eine Teilmenge der Menge der Punkte bilden, an die ein Laserpuls abgegeben wird. Dies ist in Figur 10 veranschaulicht, in der diejenigen Spots 6, die ein Zielpunkt 28 im Steuerdatensatz sind vorliegt, als schwarze ausgefüllte Kreise eingezeichnet sind.

Dieses Vorgehen hat zudem den Vorteil, daß die Maximalfrequenz $f_S$, die bei der Ansteuerung des Scanners auftritt, sehr viel geringer sind, als die Laserpulsfrequenz $f_P$. Beispielsweise kann mit einer Ansteuerfrequenz von 20 kHz sowie einer Laserpulsfrequenz von 200 kHz gearbeitet werden. Im Ergebnis liegen somit zwischen den Zielpunkten 28, die bei der Ansteuerung des Scanners vorgegeben werden, ein oder mehrere Spots 6, auf die ebenfalls gepulste Laserstrahlung abgegeben wird.

Es erfolgt also nicht nur eine Abgabe von gepulster Laserstrahlung, während sich die Scanner in einem Verstellvorgang befinden, beispielsweise während die Galvanometerspiegel sich bewegen, sondern Laserpulse werden von den Scannern abgelenkt, während diese sich von einem vorgegebenen Zielpunkt zum nächsten bewegen. Um eine möglichst hohe Ablenkgeschwindigkeit zu erreichen, stellt diese Bewegung eine Schwingung dar, die bei perfekten Kreisspiralen (wie sie bei der anterioren Schnittfläche 19 auftreten) sogar eine rein sinusförmige Schwingung ist. Da auch bei der Lentikel- Fläche 20 die tatsächliche Spiralform nur- wenig- von- idealen Kreis- oder elliptischen Spiralen abweicht, können die Scanner nahe ihrer Grenzfrequenz betrieben werden, so daß die beschriebenen Bahnen, entlang derer die Spots angeordnet sind, eine sehr schnelle Schnittflächenerzeugung erlauben.

Nach der Ermittlung der Steuerdatensätze enthaltend der Zielpunkte aus den geschilderten Punktmengen, die für die Bahnkurven erhalten wurden, ist das Vorverfahren abgeschlossen, das zur Bereitstellung der entsprechenden Steuerwert oder -parameter durchgeführt wurde. Für dieses Vorverfahren ist eine menschliche Mitwirkung und insbesondere die Mitwirkung eines Arztes oder Chirurgen nicht erforderlich. Das Verfahren wird vom Steuergerät 12 ohne Tätigkeit eines Mediziners ausgeführt. Dessen Anwesenheit ist erst für den nachgelagerten chirurgischen Eingriff erforderlich.

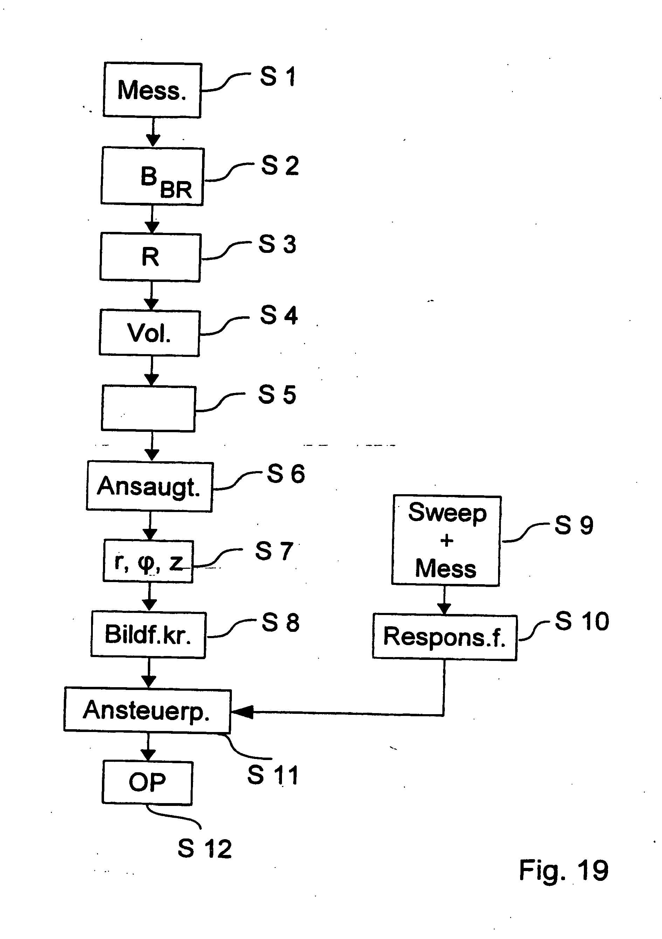

Der Ablauf des Verfahrens zur Vorbereitung des Gerätes 1 auf den Einsatz bei einer augenchirurgischen Fehlsichtigkeitsoperation ist schematisch in Figur 19 zusammengefaßt. In einem Schritt S1 erfolgt die Vermessung des Auges 3. Dabei werden für die beim Patienten 4 vorliegenden Fehlsichtigkeit Korrekturparameter erhalten, wie sie z.B. für herkömmliche Brillen üblich sind. Die in Schritt S2 aufgestellten Parameter werden dann in einem Schritt S3 dazu verwendet, die zur Korrektur nötige neue Krümmung der Hornhaut 5 zu bestimmen. Ist diese Berechnung in Schritt S3 abgeschlossen, wird das Volumen in S4 bestimmt, das aus der Hornhaut entnommen werden muß. Dies geschieht üblicherweise unter Bestimmung der Lentikel-Fläche 20 sowie der Flap-Fläche 19 in einem Schritt S5. Hat man die entsprechenden Funktionsbeschreibungen dieser Flächen gewonnen, wird in Schritt S6 die Ansaugtransformation, die sich beim Ansaugen des Auges an das Kontaktglas auswirkt, berücksichtigt.

Als nächstes werden die Koordinaten der Bahnkurven ermittelt, aus denen die Schnittflächen aufgebaut werden. Dies ist schematisch in Schritt S7 durch die Parameter r, $\varphi$, z angedeutet. Am Ende des Schrittes S7 liegt ein Punkt-Muster mit den Koordinaten der Spots, auf die ein Laserstrahlungspuls einwirken soll. Die Dichte der Zielpunkte kann dabei bereits zur Vereinfachung des rechnerischen Aufwandes reduziert sein, indem nicht für jeden mit Laserstrahlung beaufschlagten Spot eine Stützstelle bei der Ansteuerung der Scanner angegeben wird.

Nachfolgend wird die derart erhaltene Koordinatenmenge in Schritt S8 zur Berücksichtigung des Fokuslagenfehlers nochmals transformiert. In einem Schritt S11 werden dann die eigentlichen Ansteuerparameter ermittelt, wobei eine Response-Funktion eingeht, in einem Schritt S10 aus einer zuvorigen Messung (Schritt S9) des Amplituden- und Frequenzverhaltens der Scanner erhalten wurde.

Mit den derart ermittelten Ansteuerparametern wird dann in Schritt S12 die eigentliche Operation durchgeführt, bei der nun vorzugsweise zwischen den einzelnen Stützstellen, die bei der Ansteuerung des Scanners zugrunde liegen, zusätzliche Spots mit Laserstrahlungspulsen beaufschlagt werden.

**Patentansprüche**

**1.** Behandlungsvorrichtung zur operativen Fehlsichtigkeitskorrektur eines Auges (3) eines Patienten (4), wobei die Behandlungsvorrichtung (1) aufweist

- ein Kontaktglas (25), das eine gekrümmte Kontaktglasunterseite (26) zum Aufsetzen auf eine Hornhautvorderfläche (15) des Auge (3) und um der Hornhautvorderfläche (15) eine definierte Krümmung zu verleihen aufweist und das zum Halten des Auges (3) in einer vordefinierten räumlichen Lage gegenüber der Behandlungsvorrichtung (1) ausgebildet ist, und
- eine von einer Steuereinrichtung (12) gesteuerte Lasereinrichtung (L) , welche durch Einstrahlen von Laserstrahlung (2) Hornhaut-Gewebe trennt,
- wobei die Steuereinrichtung (12) die Lasereinrichtung (L) zur Fokussierung der Laserstrahlung (2) in die Hornhaut (5) auf in einem Muster in der Hornhaut (5) liegende Zielpunkte (28) ansteuert und das Muster so wählt, daß es in der Grenzfläche eines Volumens (18) in der Hornhaut (5) liegt, dessen Entfernung aus der Hornhaut (5) die gewünschte Fehlsichtigkeitskorrektur bewirkt,

wobei die Steuereinrichtung das Muster so wählt, daß die Grenzfläche ein Volumen umschreibt, das derart ausgebildet ist, daß die um das Volumen (18) verminderte Hornhaut (5) einen Krümmungsradius $R_{CV}{}^*$ hat, der folgender Gleichung genügt:

$$R_{CV}{}^* = 1 / ( (1/R_{CV}) + B_{BR} / ( (n_c\text{-}1) (1 - d_{HS} \cdot B_{BR}))) + F,$$

wobei $R_{CV}$ der Krümmungsradius der Hornhaut (5) vor Entfernung des Volumens (18), $n_c$ die Brechkraft des Materials der Hornhaut (5), F ein Faktor, $B_{BR}$ die Brechkraft einer für die Fehlsichtigkeitskorrektur tauglichen Brille (17), sowie $d_{HS}$ der Abstand ist, in dem die Brille (17) mit der Brechkraft $B_{BR}$ vor dem Hornhautscheitel liegen müßte, um die gewünschte Fehlsichtigkeitskorrektur mittels der Brille (17) zu erreichen wobei die Steuereinrichtung mittels der Gleichung die Grenzfläche ermittelt, das Muster der Zielpunkte in der Grenzfläche liegend festlegt, und auf Basis des Musters eine Bahnkurve fest legt, entlang der der Laserstrahl verstellt wird, um eine Schnittfläche in der Grenzfläche auszubilden, wobei

2. Vorrichtung nach Anspruch 1, wobei das Volumen (18) durch eine Grenzfläche begrenzt ist, die in eine anteriore und eine posteriore Teilfläche (19, 20) aufweist, wobei die anteriore Teilfläche (19) so gewählt wird, daß sie in konstantem Abstand $d_F$ zur Hornhautvorderfläche (15) liegt.

$$F = (1 - 1/n_c) \cdot (d_C{}^* - d_C)$$

gilt, wobei $d_C$ bzw. $d_C{}^*$ die Dicke der Hornhaut (5, 5*) vor bzw. nach Entfernung des Volumens (18) bezeichnet und der Radius $R_{CV}{}^*$ iterativ berechnet ist, indem bei jedem Iterationsschritt aus der Differenz ($R_{CV}{}^*$-$R_{CV}$) auf eine Dickenänderung ($d_C{}^*$ - $d_C$) geschlossen und das entsprechend daraus erhaltene Ergebnis für die Dickenänderung im nächsten Iterationsschritt bei der Berechnung von $R_{CV}{}^*$ angewendet wird.

3. Vorrichtung nach einem der obigen Ansprüche, wobei zur Berücksichtigung einer zylindrischen Fehlsichtigkeit des Auges (2) der Radius $R_{CV}{}^*$ eine Funktion des Zylinderwinkels $\varphi$ ist, also $R_{CV}{}^*(\varphi) = 1/((1/R_{CV}(\varphi)) + B_{BR}(\varphi) / ( ( n_c - 1 ) (1 - d_{HS} \cdot B_{BR} (\varphi) ))) + F$ gilt.

4. Vorrichtung nach einem der obigen Ansprüche, wobei die Steuereinrichtung für das Muster eine Verforrnung der Hornhaut, die sich aufgrund des Anpressens des Kontaktglases einstellt, festlegt und durch eine Koordinatentransformation berücksichtigt.

5. Vorrichtung nach Anspruch 1, wobei die posteriore Teilfläche (20) so gewählt wird, daß sie gekrümmt ist und einen Krümmungsradius $R_L = R_{CV}{}^* - d_F$ hat.

6. Vorrichtung nach Anspruch 5, wobei die posteriore Teilfläche (20) in Zylinderkoordinaten (z, r, $\varphi$), deren Ursprung am Durchtrittspunkt der Sehachse (OA) durch die Hornhautvorderfläche (15) liegt, der Gleichung

$$z_L(r,\varphi) = R_L(\varphi) - (R_L{}^2(\varphi) - r^2)^{1/2} + d_L + d_F$$

genügt, wobei $d_L$ eine vorzugsweise im Zentrum des Volumens (18) gegebene Mindestdicke des zu entfernenden Volumens (18) festlegt.

7. Verfahren zum Erzeugen von Steuerdaten für eine Lasereinrichtung (L) einer Behandlungsvorrichtung (1) zur operativen Fehlsichtigkeitskorrektur eines Auges (3) eines Patienten (4), welche durch Einstrahlen fokussierter Laserstrahlung (2) Hornhaut-Gewebe trennt, und ein Kontaktglas (25), das ein gekrümmte Kontaktglasunterseite (26) zum Aufsetzen auf eine Hornhautvorderfläche (15) des Auge (3), und um der Hornhautvorderfläche (15) eine definierte Krümmung zu verleihen, aufweist, und das zum Halten des Auges (3) in einer vordefinierten räumlichen Lage gegenüber der Behandlungsvorrichtung (1) ausgebildet ist, wobei die Steuerdaten im Betrieb der Behandlungsvorrichtung (1) der Lasereinrichtung (L) Zielpunkte (28) für die fokussierte Laserstrahlung (2) vorgeben, die in einem Muster in der Hornhaut (5) so liegen, daß sie eine Grenzfläche aus Volumen (18) in der Hornhaut (5) definieren, dessen Entfernung aus der Hornhaut (5) die gewünschte Fehlsichtigkeitskorrektur bewirkt, wobei die Steuerdaten so ermittelt werden, daß die Grenzfläche ein Volumen umschreibt, das derart ausgebildet ist, daß die um das Volumen (18) verminderte Hornhaut (5) einen Krümmungsradius $R_{CV}{}^*$ hat, der folgender Gleichung genügt:

$$R_{CV}{}^* = 1 / ( (1/R_{CV}) + B_{BR} / ( (n_c\text{-}1) (1 - d_{HS} \cdot B_{BR}))) + F,$$

wobei $R_{CV}$ der Krümmungsradius der Hornhaut (5) vor Entfernung des Volumens (18), $n_c$ die Brechkraft des Materials der Hornhaut (5), F ein Faktor ist, $B_{BR}$ die Brechkraft einer für die Fehlsichtigkeitskorrektur tauglichen Brille (17), sowie $d_{HS}$ der Abstand ist, in dem die Brille (17) mit der Brechkraft $B_{BR}$ vor dem Hornhautscheitel liegen müßte, um die gewünschte Fehlsichtigkeitskorrektur mittels der Brille (17) zu erreichen, wobei die Grenzfläche mittels der Gleichung ermittelt werden und die Steuerdaten das Muster der Zielpunkte in der Grenzfläche liegend festlegen, wobei auf Basis des Musters eine Bahnkurve festgelegt wird, entlang der der Laserstrahl verstellbar ist, um ein Schnittfläche in der Grenzfläche auszubilden, und

wobei die Grenzfläche eine anteriore und eine posteriore Teilfläche (19, 20) aufweist, wobei die anteriore Teilfläche (19) in konstantem Abstand $d_F$ zur Hornhautvorderfläche (15) liegt.

8. Verfahren nach dem obigen Verfahrensanspruche, wobei

$$F = (1 - 1/n_c) \cdot (d_C{}^* - d_C)$$

gilt, wobei $d_C$ bzw. $d_C{}^*$ die Dicke der Hornhaut (5, 5*) vor bzw. nach Entfernung des Volumens (18) bezeichnet und der Radius $R_{CV}{}^*$ iterativ berechnet wird, indem bei jedem Iterationsschritt aus der Differenz ($R_{CV}{}^* - R_{CV}$) auf eine Dickenänderung ($d_C{}^* - d_C$) geschlossen wird und das entsprechende daraus erhaltene Ergebnis für die Dickenänderung bei der Berechnung von $R_{CV}{}^*$ im nächsten Iterationsschritt angewendet wird.

9. Verfahren nach einem der obigen Verfahrensansprüche, **dadurch gekennzeichnet, daß** zur Berücksichtigung einer zylindrischen Fehlsichtigkeit des Auges (2) der Radius $R_{CV}{}^*$ als Funktion des Zylinderwinkels $\varphi$ gewählt wird, also $R_{CV}{}^*(\varphi) = 1 / ((1 / R_{CV}(\varphi)) + B_{BR}(\varphi) / ((n_c-1) (1 - d_{HS} \cdot B_{BR}(\varphi) ))) + F$ gilt.

10. Verfahren nach einem der obigen Verfahrensansprüche, wobei für das Muster eine Verformung der Hornhaut, die sich aufgrund des Anpressens des Kontaktglases einstellt, durch eine Koordinatentransformation Berücksichtigt wird.

11. Verfahren nach Anspruch 10, wobei die posteriore Teilfläche gekrümmt ist und einen Krümmungsradius $R_L = R_{CV}{}^* - d_F$ hat.

12. Verfahren nach Anspruch 10, wobei die posteriore Teilfläche (20) in Zylinderkoordinaten (z, r, $\varphi$), deren Ursprung am Durchtrittspunkt der Sehachse (OA) durch die Hornhautvorderfläche (15) liegt, der Gleichung

$$z_L(r,\varphi) = R_L(\varphi) - (R_L{}^2(\varphi) - r^2)^{1/2} + d_L + d_F$$

genügt, wobei durch $d_L$ eine Mindestdicke des zu entfernenden Volumens (18) festgelegt wird.

**Claims**

1. Treatment apparatus for surgical correction of defective eyesight in an eye (3) of a patient (4), wherein the treatment apparatus (1) comprises:

    - a contact glass (25) with a curved contact glass underneath side (26) so it can be placed on an anterior corneal surface (15) of the eye (3) in order to impart a defined curvature to the anterior corneal surface (15) and is designed to hold the eye (3) in a predefined spatial position relative to the treatment apparatus (1), and
    - a laser device (L) controlled by a control device (12), which separates corneal tissue by irradiation with laser radiation (2),
    - wherein the control device (12) activates the laser device (L) for focusing the laser radiation (2) onto the cornea (5) on target points (28) lying in a pattern in the cornea (5) and selects the pattern so that it lies in the boundary surface of a volume (18) in the cornea (5), whose removal from the cornea (5) produces the desired defective eyesight correction,
    - wherein the control device selects the pattern so that the boundary surface circumscribes a volume that is formed in such a way that the cornea (5) minus the volume (18) has a radius of curvature $R_{cv}{}^*$ that satisfies the following equation:

$$R_{cv}* = 1 / ((1/R_{cv}) + B_{BR}/ ( (n_c\text{-}1) (1\text{-}d_{HS} \cdot B_{BR}))) + F,$$

where $R_{cv}$ is the radius of curvature of the cornea (5) prior to removal of the volume (18), $n_c$ is the refractive power of the material of the cornea (5), F is a factor, $B_{BR}$ is the refractive power of spectacles (17) suitable for correcting the defective eyesight, and $d_{HS}$ is the distance at which the spectacles (17) with the refractive power $B_{BR}$ should be located anterior of the corneal vertex in order to achieve the desired defective eyesight correction by means of the spectacles (17), wherein the control device determines the boundary surface by means of the equation, defines the pattern of the target points lying in the boundary surface, and on the basis of the pattern specifies a path curve, along which the laser beam is adjusted, in order to form an interface in the boundary surface, wherein the volume (18) is bounded by a boundary surface that has anterior and posterior partial surfaces (19, 20), wherein the anterior partial surface (19) is chosen so that it lies at a constant distance $d_F$ from the anterior corneal surface (15).

2. Apparatus according to claim 1, wherein

$$F = (1 - 1/n_c) \bullet (d_c* - d_c)$$

applies, wherein $d_c$ and $d_c*$ denote the thickness of the cornea (5, 5*) before and after removal of the volume (18) and the radius $R_{CV}*$ is calculated iteratively, wherein with each iteration step a change in thickness $(d_c* - d_c)$ is obtained from the difference $(R_{CV}* - R_{CV})$ and the correspondingly obtained result is used for the change in thickness in the next iteration step in the calculation of $R_{CV}*$.

3. Apparatus according to one of the above claims, wherein in order to take account of a cylindrical defective eyesight of the eye (2) the radius $R_{CV}*$ is a function of the cylindrical angle $\omega$, i.e. $R_{CV}*(\omega) = 1 / ((1 / R_{CV}(\omega) + B_{BR}(\omega) / ((n_c\text{-}1) (1 - d_{HS} \cdot B_{BR}(\omega)))) + F$.

4. Apparatus according to one of the above claims, wherein the control device specifies and takes into account by means of a coordinate transformation the pattern of a deformation of the cornea that occurs on account of the impression of the contact glass.

5. Apparatus according to claim 1, wherein the posterior partial surface (20) is chosen so that it is curved and has a radius of curvature $R_L = R_{CV}* - d_F$.

6. Apparatus according to claim 5, wherein the posterior partial surface (20) in cylindrical coordinates (z, r, $\omega$), whose origin lies at the point where the axis of vision (OA) passes through the anterior corneal surface (15), satisfies the equation

$$Z_{L\_}(r,\omega) = R_L(\omega) - (R_L^2(\omega) - r^2)^{1/2} + d_L + d_F$$

where $d_L$ defines a minimum thickness of the volume (18) to be removed, preferably specified in the centre of the volume (18) .

7. Method for generating control data for a laser device (L) of a treatment apparatus (1) for surgical correction of defective eyesight in an eye (3) of a patient (4), which by irradiation with focused laser radiation (2) separates corneal tissue, and has a contact glass (25), with a curved contact glass underneath side (26) so it can be placed on the anterior corneal surface (15) of the eye (3), in order to impart a defined curvature to the anterior corneal surface (15), and which is formed to hold the eye (3) in a predefined spatial position relative to the treatment apparatus (1), wherein the control data in the operation of the treatment apparatus (1) of the laser device (L) specify target points (28) for the focused laser radiation (2) that lie in a pattern in the cornea (5) so that they define a boundary surface of volume (18) in the cornea (5), whose removal from the cornea (5) produces the desired defective eyesight correction, wherein the control data are determined so that the boundary surface circumscribes a volume that is formed in such a way that the cornea (5) minus the volume (18) has a radius of curvature $R_{cv}*$ that satisfies the following equation:

$$R_{cv}^* = 1 / ((1/R_{cv}) + B_{BR}/ ( (n_c\text{-}1) (1\text{-}d_{HS}{\cdot}B_{BR}))) + F,$$

wherein $R_{cv}$ is the radius of curvature of the cornea (5) prior to removal of the volume (18), $n_c$ is the refractive power of the material of the cornea (5), F is a factor, $B_{BR}$ is the refractive power of spectacles (17) suitable for correcting the defective sight, and $d_{HS}$ is the distance at which the spectacles (17) with the refractive power $B_{BR}$ should be located anterior of the corneal vertex in order to achieve the desired defective eyesight correction by means of the spectacles (17), wherein the boundary surface is determined by means of the equation and the control data specify the pattern of the target points lying in the boundary surface, wherein on the basis of the pattern a path curve is defined, along which the laser beam can be adjusted, in order to form an interface in the boundary surface, and wherein the boundary surface has an anterior and a posterior partial surface (19, 20), wherein the anterior partial surface (19) lies at a constant distance $d_F$ from the anterior corneal surface (15).

8. Method according to the above method claim, wherein

$$F = (1 - 1/n_c) \bullet (d_c^* - dc)$$

wherein $d_c$ and $d_c^*$ denote the thickness of the cornea (5, 5*) before and after removal of the volume (18) and the radius $R_{CV}^*$ is iteratively calculated, in which with each iteration step a thickness change $(d_c^*\text{-} dc)$ is determined from the difference $(R_{CV}^*\text{-}R_{CV})$ and the corresponding result obtained therefrom is used for the change of thickness in the calculation of $R_{CV}^*$ in the next iteration step.

9. Method according to one of the above method claims, **characterised in that** in order to take into account a cylindrical defective eyesight of the eye (2), the radius $R_{CV}^*$ is chosen as a function of the cylindrical angle ($\omega$), i.e. $R_{CV}^* (\omega)$ = 1 / ((1 / $R_{CV}(\omega)$ + $B_{BR} (\omega)$ / ((n$_c$ - 1) (1-$d_{HS}.B_{BR}(\omega)$ ))) + F.

10. Method according to one of the above method claims wherein for the pattern a deformation of the cornea, that occurs on account of the impression of the contact glass, is taken into account by a coordinate transformation.

11. Method according to claim 10, wherein the posterior partial surface is curved and has a radius of curvature $R_L$ = $R_{CV}^*$ - $d_F$.

12. Method according to claim 10, wherein the posterior partial surface (20) in cylindrical coordinates (z, r, $\omega$), whose origin lies at the point where the axis of vision (OA) passes through the anterior corneal surface (15), satisfies the equation

$$Z_L(r,\omega) = R_L(\omega) - (R_L^2(\omega) - r^2)^{1/2} + d_L + d_F$$

wherein a minimum thickness of the volume (18) to be removed is specified by $d_L$.

**Revendications**

1. Dispositif de traitement de correction d'un défaut visuel de l'oeil (3) d'un patient (4) par opération, étant entendu que le dispositif de traitement (1) présente :

   - un verre de contact (25) qui présente un côté inférieur courbé du verre de contact (26) destiné à être posé sur une surface avant d'une cornée (15) de l'oeil (3) et à conférer une courbure définie à la surface avant de la cornée (15) et qui est conçu pour maintenir l'oeil (3) dans une position dimensionnelle prédéfinie par rapport au dispositif de traitement (1) ; et
   - un dispositif laser (L) commandé par un dispositif de commande (12) qui sectionne des tissus de cornée par projection d'un rayonnement laser (2) ;
   - étant entendu que le dispositif de commande (12) commande le dispositif laser (L) de façon à focaliser le rayonnement laser (2) dans la cornée (5) sur des points cibles (28) se trouvant dans un dessin dans la cornée (5) et choisit le dessin de telle sorte qu'il se trouve dans l'interface d'un volume (18) dans la cornée (5) dont l'ablation de la cornée (5) produit la correction souhaitée du défaut visuel ;

étant entendu que le dispositif de commande choisit le dessin de telle sorte que l'interface délimite un volume qui est formé de telle sorte que la cornée (5) réduite du volume (18) présente un rayon de courbure $R_{CV}{}^*$ qui satisfait à l'équation suivante :

$$R_{CV}{}^* = 1 \ / \ (\ (1/R_{CV}) + B_{BR} \ / \ (\ (n_c\text{-}1)\ (1 - d_{HS} \cdot B_{BR}))) + F,$$

où $R_{CV}$ désigne le rayon de courbure de la cornée (5) avant l'ablation du volume (18), $n_c$ la vergence du matériau de la cornée (5), F un facteur, $B_{BR}$ la vergence de lunettes (17) appropriées pour la correction du défaut visuel et $d_{HS}$ la distance à laquelle les lunettes (17) ayant la vergence $B_{BR}$ devraient se trouver à l'avant du sommet de la cornée pour obtenir la correction souhaitée du défaut visuel au moyen des lunettes (17),

étant entendu que le dispositif de commande, au moyen de l'équation, détermine l'interface et établit le dessin des points cibles se trouvant dans l'interface, et sur la base du dessin, établit une courbe de trajectoire le long de laquelle le rayonnement laser est réglé afin de constituer une surface de coupe dans l'interface, étant entendu que le volume (18) est délimité par une interface qui présente une surface partielle antérieure et postérieure (19, 20), étant entendu que la surface partielle antérieure (19) est choisie de telle sorte qu'elle se trouve à une distance $d_F$ constante par rapport à la surface avant de la cornée de l'oeil (15).

2. Dispositif selon la revendication 1, dans lequel :

$$F = (1 - 1/n_c) \cdot (d_c{}^* - d_c)$$

étant entendu que $d_c$ et $d_c{}^*$ désignent l'épaisseur de la cornée (5, 5*) avant et après l'ablation du volume (18) et que le rayon $R_{CV}{}^*$ est calculé par itération en ce qu'à chaque étape de l'itération, une modification de l'épaisseur $(d_c{}^*\text{-}d_c)$ est déduite à partir de la différence $(R_{CV}{}^* - R_{CV})$ et le résultat ainsi obtenu en conséquence pour la modification de l'épaisseur est appliqué à l'étape suivante de l'itération dans le cadre du calcul de $R_{CV}{}^*$.

3. Dispositif selon l'une des revendications précédentes, dans lequel afin de prendre en considération un défaut visuel cylindrique de l'oeil (2), le rayon $R_{CV}{}^*$ est une fonction de l'angle du cylindre $\varphi$, et par conséquent, $R_{CV}{}^*(\varphi) = 1/((1/R_{CV}(\varphi)) + B_{BR}(\varphi) \ / \ ((n_c - 1)\ (1 - d_{HS} \cdot B_{BR}(\varphi)\ ))) + F$.

4. Dispositif selon l'une des revendications précédentes, dans lequel le dispositif de commande établit et prend en considération pour le dessin, au moyen d'une transformation des coordonnées, une déformation de la cornée qui résulte de la pression du verre de contact.

5. Dispositif selon la revendication 1, dans lequel la surface partielle postérieure (20) est choisie de telle sorte qu'elle est courbée et présente un rayon de courbure $R_L = R_{CV}{}^* - d_F$.

6. Dispositif selon la revendication 5, dans lequel la surface partielle postérieure (20) satisfait, dans des coordonnées de cylindre $(z, r, \varphi)$ dont l'origine se trouve au point de passage de l'axe visuel (OA) à travers la surface avant de la cornée (15), à l'équation

$$z_L\ (r,\varphi) = R_L\ (\varphi) - (R_L{}^2(\varphi) - r^2)^{1/2} + d_L + d_F$$

étant entendu que $d_L$ établit une épaisseur minimale du volume (18) à enlever située de préférence au centre du volume (18).

7. Procédé de production de données de commande pour un dispositif laser (L) d'un dispositif de traitement (1) de correction d'un défaut visuel d'un oeil (3) d'un patient (4) qui sectionne des tissus de cornée par projection d'un rayonnement laser (2) focalisé et un verre de contact (25) qui présente un côté inférieur courbé du verre de contact (26) destiné à être posé sur une surface avant d'une cornée (15) de l'oeil (3), et à conférer une courbure définie à la surface avant de la cornée (15), et qui est conçu pour maintenir l'oeil (3) dans une position dimensionnelle prédéfinie par rapport au dispositif de traitement (1), étant entendu que dans le fonctionnement du dispositif de traitement (1), les données de commande prescrivent au dispositif laser (L) des points cibles (28) pour le rayonnement laser (2) focalisé qui se situent dans un dessin dans la cornée (5) de telle sorte qu'ils définissent une interface d'un volume (18) dans la cornée (5) dont l'ablation de la cornée (5) produit la correction souhaitée du défaut visuel ; étant entendu que les données de commande sont déterminées en ce que l'interface délimite un volume qui est formé de telle sorte que la cornée (5) réduite du volume (18) présente un rayon de courbure $R_{CV}{}^*$ qui satisfait à l'équation suivante :

$$R_{CV}{}^* = 1 \,/\, (\,(1/R_{CV}) + B_{BR} \,/\, (\,(n_c-1)\,(1 - d_{HS} \cdot B_{BR})))) + F,$$

où $R_{CV}$ désigne le rayon de courbure de la cornée (5) avant l'ablation du volume (18), $n_c$ la vergence du matériau de la cornée (5), F un facteur, $B_{BR}$ la vergence de lunettes (17) appropriées pour la correction du défaut visuel et $d_{HS}$ la distance à laquelle les lunettes (17) ayant la vergence $B_{BR}$ devraient se trouver à l'avant du sommet de la cornée pour obtenir la correction souhaitée du défaut visuel au moyen des lunettes (17) ; étant entendu que l'interface est déterminée au moyen de l'équation et que les données de commande établissent le dessin des points cibles dans l'interface, étant entendu une courbe de trajectoire le long de laquelle le rayonnement laser peut être réglé afin de constituer une surface de coupe dans l'interface est établie sur la base du dessin ; et étant entendu que l'interface présente une surface partielle antérieure et postérieure (19, 20), étant entendu que la surface partielle antérieure (19) se trouve à une distance $d_F$ constante par rapport à la surface avant de la cornée de l'oeil (15).

8. Procédé selon la revendication précédente, dans lequel :

$$F = (1 - 1/n_c) \cdot (d_c{}^* - d_c)$$

étant entendu que $d_c$ et $d_c{}^*$ désignent l'épaisseur de la cornée (5, 5*) avant et après l'ablation du volume (18) et que le rayon $R_{CV}{}^*$ est calculé par itération en ce qu'à chaque étape de l'itération, une modification de l'épaisseur $(d_c{}^*-d_c)$ est déduite à partir de la différence $(R_{CV}{}^* - R_{CV})$ et le résultat ainsi obtenu en conséquence pour la modification de l'épaisseur est appliqué à l'étape suivante de l'itération dans le cadre du calcul de $R_{CV}{}^*$.

9. Procédé selon l'une des revendications précédentes, **caractérisé en ce qu'**afin de prendre en considération un défaut visuel cylindrique de l'oeil (2), le rayon $R_{CV}{}^*$ est choisi comme fonction de l'angle du cylindre $\varphi$, et par conséquent, $R_{CV}{}^*(\varphi) = 1/((1/ R_{CV}(\varphi)) + B_{BR}(\varphi) / ((n_c - 1) (1 - d_{HS} \cdot B_{BR}(\varphi)))) + F$.

10. Procédé selon l'une des revendications précédentes, dans lequel pour le dessin, une déformation de la cornée qui résulte de la pression du verre de contact est prise en considération au moyen d'une transformation des coordonnées.

11. Procédé selon la revendication 10, dans lequel la surface partielle postérieure est courbée et présente un rayon de courbure $R_L = R_{CV}{}^* - d_F$.

12. Dispositif selon la revendication 10, dans lequel la surface partielle postérieure (20) satisfait, dans des coordonnées de cylindre (z, r, $\varphi$) dont l'origine se trouve au point de passage de l'axe visuel (OA) à travers la surface avant de la cornée (15), à l'équation

$$z_L\,(r,\varphi) = R_L\,(\varphi) - (R_L{}^2(\varphi) - r^2)^{1/2} + d_L + d_F$$

étant entendu que $d_L$ établit une épaisseur minimale du volume (18) à enlever.

# FIG 1

# FIG 2

Fig. 5

Fig. 6

Fig. 1a

FIG 3

Fig. 7

Fig.4

a)

b)

c)

FIG 8

**FIG 9**

24

Fig 10

$d_S$

24

28

6

$d_T$

Fig. 11

24

$a$

$b$

$d_{T_b}$

$d_{T_a}$

**FIG 12**

Fig. 13

Fig. 14

Fig. 15

Fig. 18

Fig. 16

Fig. 17

30

Fig. 19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5984916 A
- US 2004243112 A1
- WO 2005011547 A1
- US 6110166 A
- WO 2005092172 A
- WO 9611655 A
- EP 1159986 A1

- US 5549632 A
- DE 69500997 T2
- WO 2004032810 A2
- WO 2005011546 A
- WO 2005011545 A
- WO 2005048895 A
- WO 2003002008 A